# EUROPÄISCHE PATENTSCHRIFT

(11) **EP 2 316 025 B1**
(45) Veröffentlichungstag und Bekanntmachung des Hinweises auf die Patenterteilung: **28.11.2012**
(21) Anmeldenummer: 09777865.8
(22) Anmeldetag: 13.08.2009
(51) Int. Cl.: G01N 33/574, G01N 33/50

(54) **MITTEL ZUM NACHWEIS UND ZUR BEHANDLUNG VON PROSTATAZELLEN**
MEANS FOR THE DETECTION AND TREATMENT OF PROSTATE CELLS
MOYEN DE DÉTECTION ET DE TRAITEMENT DE CELLULES PROSTATIQUES

(30) Priorität: 13.08.2008 DE 102008039417
(43) Veröffentlichungstag der Anmeldung: 04.05.2011
(73) Patentinhaber: Eberhard-Karls-Universität Tübingen Universitätsklinikum, 72076 Tübingen (DE)
(72) Erfinder: HECKL, Stefan, 72076 Tübingen (DE)
(74) Vertreter: Witte, Weller & Partner
(86) Internationale Anmeldenummer: PCT/EP2009/005882
(87) Internationale Veröffentlichungsnummer: WO 2010/017986

(56) Entgegenhaltungen:
- WO-A-2004/033496
- DE-A1-102006 008 074
- JACQUIER VALERIE ET AL: "Characterization of an extended receptive ligand repertoire of the human olfactory receptor OR17-40 comprising structurally related compounds" JOURNAL OF NEUROCHEMISTRY, Bd. 97, Nr. 2, April 2006 (2006-04), Seiten 537-544, XP002549462 ISSN: 0022-3042
- XU L L ET AL: "Quantitiative expression profile of PSGR in prostate cancer" PROSTATE CANCER AND PROSTATIC DISEASES, Bd. 9, 2006, Seiten 56-61, XP009123910 England ISSN: 0022-3042
- SPEHR MARC ET AL: "Identification of a testicular odorant receptor mediating human sperm chemotaxis" SCIENCE, Bd. 299, März 2003 (2003-03), Seiten 2054-2058, XP002268735 ISSN: 0022-3042

## Beschreibung

Die vorliegende Erfindung betrifft ein Mittel zur Identifizierung und zur Behandlung von Prostatazellen sowie Verfahren zur diagnostischen und therapeutischen Behandlung eines Lebewesens, in dem das erfindungsgemäße Mittel eingesetzt wird.

Mittel zur Identifizierung und zur Behandlung von Prostatazellen sind im Stand der Technik allgemein bekannt.

Das Prostatakarzinom ist eine bösartige Tumorerkrankung, die von dem Drüsengewebe der Vorsteherdrüse bzw. Prostata ausgeht. In Deutschland sterben knapp drei von 100 Männern an Prostatakarzinom. Damit gehört das Prostatakarzinom zu den häufigsten Krebserkrankungen des Mannes. Es ist innerhalb der Gruppe der an Krebs gestorbenen Männer für etwa 10 % der Todesfälle verantwortlich und stellt damit die dritthäufigste tödliche Krebserkrankung nach Lungen- und Darmkrebs dar.

Voraussetzung für eine therapeutische Behandlung des Prostatakarzinoms ist eine zuverlässige Diagnose. Entscheidend ist vor allem, ob bei einem betroffenen Patienten bereits eine Lymphknotenmetastase vorliegt; Messing E.M. et al. (1999), Immediate hormonal therapy compared with observation after radical prostatectomy and pelvic lymphadenectomy in men with node-positive prostate cancer. N. Engl. J. Med.; 341 (24): 1781-1788; Walsh P.C. (2002), Surgery and the reduction of mortality from prostate cancer. N. Engl. J. Med.; 347 (11): 839-840.

Die Identifizierung bzw. Demarkation von Prostatakarzinommetastasen in Lymphknoten erfolgt als "Negativdarstellung" mittels der Magnetresonanztomographie (MRT). Dazu werden Eisenpartikel intravenös injiziert; vgl. Harisinghani M.G. et al. (2006), Ferumoxtran-10-enhanced MR lymphangiography: does contrast-enhanced imaging alone suffice for accurate lymph node characterization? Am. J. Roentgenol.; 186 (1): 144-8. Die Eisenpartikel werden durch das Retikuloendotheliale System (RES) des Lymphknotens aufgenommen, nicht aber von der Prostatakarzinommetastase selbst, da an dieser Stelle das RES verdrängt ist. In den T2-gewichteten MRT-Aufnahmen stellt sich das RES aufgrund der aufgenommenen Eisenpartikel dunkel dar, die Prostatakarzinommetastase aber hell.

Allerdings kommt es auch in den Bereichen von Entzündungsprozessen, Fetteinlagerungen und Fibrosen ebenfalls zu einer Verdrängung des RES, so dass sich auch diese Bereiche in der Magnetresonanztomographie hell darstellen und als Metastasenähnliche Erscheinungen wahrgenommen werden. Ferner kann mit dieser Methode nicht zwischen Metastasen verschiedener Ursprünge, d.h. Prostata, Kolon, Blase etc., unterschieden werden, da mit dieser Methode nur das RES und nicht die Krebszellen selbst dargestellt werden.

Vor diesem Hintergrund liegt der Erfindung die Aufgabe zugrunde, ein neues Mittel bereitzustellen, mit dem Prostatazellen, insbesondere Prostatakarzinomzellen sowie solche, die sich in den Lymphknoten befinden, zuverlässig und "positiv" identifiziert werden können.

Diese Aufgabe wird durch die Bereitstellung eines solchen Mittels gelöst, das einen Liganden des Maiglöckchenduftrezeptors (OR17-4), vorzugsweise der humanen Variante (hOR17-4) hiervon, aufweist.

Diese Erkenntnis war überraschend. So wurde bislang angenommen, dass Geruchsrezeptoren vorwiegend in der Nase vorkommen. Lediglich vereinzelt wurde das Vorkommen von Geruchsrezeptoren auch in anderen Geweben beschrieben.

So beschreiben bspw. Spehr et al. (2003), Identification of a testicular odorant receptor mediating human sperm chemotaxis, Science Vol. 299, Seiten 2054-2058, und Spehr et al. (2004), Dual capacity of a human olfactory receptor, Curr. Biol. Vol. 14, No. 19, R832-3, die Expression des Maiglöckchenduftrezeptors hOR17-4 auf Spermien.

In der WO 2004/033496 wird deshalb vorgeschlagen, einen Liganden für den humanen Maiglöckchenduftrezeptor hOR17-4 als Kontrazeptivum oder empfängnisförderndes Arzneimittel einzusetzen.

Nur wenige Geruchsrezeptoren wurden bisher in der Prostata gefunden, wie bspw. der OR 51 E2 [Ashida et al. (2004), Molecular features of the transition from prostatic intraepithelial neoplasia (PIN) to prostate cancer: genome-wide gene-expression profiles of prostate cancers and PINs. Cancer Res. Vol. 64, Seiten 5963-5972], der Dresden-G-Protein gekoppelte Rezeptor (D-GPCR) [Weigle et al. (2004), D-GPCR: a novel putative G protein-coupled receptor overexpressed in prostate cancer and prostate. Biochemical and Biophysical Research Communications, Vol. 322, Seiten, 239-249] oder der G-Protein gekoppelte Rezeptor 95 (GPR95) [Lee et al. (2001), Discovery and mapping of ten novel G protein-coupled receptor genes. Gene, Vol. 275, Seiten 83-91]

Vor diesem Hintergrund war bereits die Erkenntnis des Erfinders, dass der Maiglöckchenduftrezeptor OR17-4 bzw. hOR17-4 in der Prostata exprimiert wird, äußerst überraschend.

Überraschend war auch, dass Prostatakarzinomzellen den Maiglöckchenduftrezeptor OR17-4 bzw. hOR17-4 besonders stark exprimieren. Mit dem erfindungsgemäßen Mittel können deshalb nicht nur Prostatazellen identifiziert werden, es ist darüber hinaus eine Unterscheidung zwischen gesunden und entarteten Prostatazellen möglich. Eine entartete Prostatazelle bindet wesentlich mehr des erfindungsgemäßen Mittels als eine gesunde Prostatazelle.

Wie der Erfinder feststellen konnte, bindet das erfindungsgemäße Mittel an die Zelloberfläche von Prostatazellen und wird dann in großen Mengen in das Zytoplasma aufgenommen.

Wie der Erfinder ferner feststellen konnte, wird das erfindungsgemäße Mittel, außer in die Geruchsorgane, nur in Prostatazellen aufgenommen, nicht aber in Muskel-, Hoden-, Herz-, Kolon-, Lymphknoten-, Samenbläschen-, Milz-, Haut-, Gehirn- und Lungengewebe. Bemerkenswerterweise wird das erfindungsgemäße Mittel auch nicht von anderen malignen Zellen, beispielsweise eines Kolonkarzinoms, Glioms, Osteosarkoms, Cervixkarzinoms und Lymphoms, aufgenommen.

Der Erfinder stellt somit erstmals ein Mittel bereit, mit dem hoch selektiv Prostatazellen, einschließlich Prostatakarzinomzellen, "positiv" identifiziert werden können.

Der Maiglöckchenduftrezeptor trägt die offizielle Bezeichnung OR17-4, die humane Variante wird mit hOR17-4 bezeichnet. Wegen der Nomenklatur wird auf die Publikation von Ben-Arie et al. (1994), Olfactory receptor gene cluster on human chromosome 17: possible duplication of an ancestral receptor repertoire, Hum. Mol. Genet. Vol. 3, Seiten 229-235, verwiesen. Eine synonyme Bezeichnung zu hOR17-4 lautet OR1D2. Unter dieser Bezeichnung ist die Gensequenz in der GenBank-Datenbank unter der GenBank-Zugriffsnummer NM_002548 bzw. der EMBL-Zugriffsnummer AF087917 veröffentlicht; die gesamte Gensequenz ist durch Inbezugnahme Bestandteil der vorliegenden Anmeldung. Der Maiglöckchenduftrezeptor gehört zu der Gruppe der G-Protein-gekoppelten Rezeptoren.

Unter einem "Liganden" wird eine solche Verbindung verstanden, die selektiv und spezifisch unter stringenten Bedingungen an den Maiglöckchenduftrezeptor OR17-4 bzw. hOR17-4 binden kann.

Erfindungsgemäß wird unter einem "Mittel" eine jegliche Zusammensetzung verstanden, die zumindest den Liganden des humanen Maiglöckchenduftrezeptors aufweist, gegebenenfalls aber weitere Verbindungen enthalten kann, wie beispielsweise einen detektierbaren Marker sowie diagnostisch bzw. pharmazeutisch akzeptable Träger. Derartige Träger sind im Stand der Technik umfassend offenbart, bspw. in Kibbe et al. (2000), Handbook of Pharmaceutical Excipients, 3rd Edition, American Pharmaceutical Association and Pharmaceutical Press. Der Inhalt dieser Publikation ist durch Inbezugnahme Bestandteil der vorliegenden Anmeldung. Bevorzugte Mittel sind Nachweismittel, Arzneimittel, Diagnostika und Therapeutika.

Erfindungsgemäß wird unter "Identifizierung" eine jegliche Maßnahme verstanden, die der Detektion von Prostatazellen bzw. Prostatakarzinomzellen dient.

Erfindungsgemäß wird unter "Behandlung" eine jegliche Maßnahme verstanden, durch die auf Prostatazellen, insbesondere Prostatakarzinomzellen, eingewirkt wird. Insbesondere umfasst die Behandlung die therapeutische Einwirkung auf Prostatakarzinomzellen.

Die der Erfindung zugrundeliegende Aufgabe wird hiermit vollkommen gelöst.

Dabei ist es erfindungsgemäß bevorzugt, wenn der Ligand ausgewählt ist aus der Gruppe bestehend aus: Undecanal, 3-phenyl-2-propenal (Zimtaldehyd), Octanal, Bourgeonal, Benzaldehyd, Phenylacetaldehyd, 3-Phenylpropanal, 3-Phenylbutyraldehyd, 4-Phenylbutyraldehyd, Canthoxal, Cyclamal, Floralazon, Lilial, (4-tert-butylphenoxy)acetaldehyd, p-Anisyl-Format, Piperonylacetat, Zimtalkohol, Hydrozimtaldehyd, Methylzimtaldehyd, Methyl-Hydro-Zimtaldehyd, Methylnonylaldehyd, Phenylethanol, Phenylpropanol, Methylzimtaldehyd, Isobutyraldehyd, Helional, Lyral, Methyl-Phenyl-Pentanal, Cyclosal, Foliaver, Trimethylmethan, Trifernal, Piperonal, Isovaleraldehyd, (4-Hydroxyphenyl)-Butan-2-on (Himbeerketon), Vanillin, Safrol, Heptanal, Anethol, 5-Phenylvaleraldehyd, Isopropylbenzen, 3-Phenylbutylalkohol und 3-Phenylpropionsäure.

Die vorgenannten Verbindungen sind Beispiele für Liganden des Maiglöckchenduftrezeptors OR17-4 bzw. der humanen Variante hOR17-4, die zur Herstellung des erfindungsgemäßen Mittels besonders geeignet sind. Es versteht sich, dass die Liganden modifiziert werden können, um beispielsweise eine weitere Verbindung an den Liganden zu koppeln, wie einen Marker. Bei einer geeigneten Modifizierung handelt es sich beispielsweise um das Anbringen einer Aminogruppe, z.B. um Aminoundecanal, Aminobourgeonal, Aminozimtaldehyd oder Aminobenzaldehyd etc. zu bilden. Ferner können mehrere Aminosäuren vorgesehen werden. Entsprechend modifizierte Liganden sind erfindungsgemäß mit umfasst; vgl. auch V. Jacquier et al. (2006), Journal of Neuroscience, Volume 97, Seiten 537-544 und WO 2004/033496.

Erfindungsgemäß ist es bevorzugt, wenn das Mittel ferner einen mittels bildgebender Verfahren detektierbaren Marker aufweist.

Erfindungsgemäß umfassen bildgebende Verfahren sämtliche apparative Verfahren, mit denen physiologische bzw. medizinische Befunde oder physikalische und chemische Phänomene visualisiert werden können. Zu diesen Verfahren zählen beispielsweise die Fluoreszenzmikroskopie, Magnetresonanztomographie (MRT), Radiographie, Computertomographie, Positronen-Emissions-Tomographie (PET), Szintigraphie, Single Photon Emission Computed Tomography (SPECT), Sonographie, Massenspektrometrie etc. Erfindungsgemäß wird jeder Marker umfasst, der in zumindest einem der vorgenannten Verfahren detektierbar ist.

Diese Maßnahme hat den Vorteil, dass das erfindungsgemäße Mittel als Diagnostikum bereitgestellt wird. Durch die hohe Selektivität des erfindungsgemäßen Mittels können durch diese Weiterbildung erstmals Prostatazellen bzw. Prostatakarzinomzellen selbst in den Lymphknoten positiv dargestellt werden. Dies gelingt bislang auf sehr unspezifische Art und Weise in Form einer Negativdarstellung durch die Gabe von Eisenpartikeln, bei der auch Bereiche angefärbt werden, die keine Prostatazellen enthalten.

Dabei ist es bevorzugt, wenn es sich bei dem Marker um einen solchen handelt, der mittels Fluoreszenzmikroskopie detektierbar ist und vorzugsweise ausgewählt ist aus: Fluorescein-Isothiocyanat (FITC), Rhodamin, Rhodamin-Isothiocyanat (RITC), Sulforhodamin, Dansylchlorid, Fluorescamin, grünfluoreszierendes Protein (GFP), Ethidiumbromid, 4',6-Diamidino-2-phenylindol (DAPI), Coumarin, Luciferase, Phycoerythrin (PE), Cy2, Cy3.5, Cy5, Cy 7, Texas Red, Alexa Fluor, Fluor X, Red 613, BODIPY-FL, TRITC, DS Red, GFP, DS Red.

Bei den vorgenannten Markern handelt es sich um solche, die sich in der molekularen Diagnostik bewährt haben und mittels Standardverfahren an den Liganden gebunden werden können. Dazu kann es erforderlich sein, dass der detektierbare Marker modifiziert wird, indem beispielsweise Schwefel- und/oder Aminogruppen vorgesehen werden, um z.B. eine Sulforhodaminsulfonamidoverbindung oder FITC-βAla-Verbindungen zu erhalten. Derartig modifizierte Marker sind erfindungsgemäß umfasst.

Bei dem Marker kann es sich ferner um einen mittels Magnetresonanztomographie (MRT) detektierbaren Marker handeln, vorzugsweise um einen Komplexbildner für Metalle mit einem komplexierten Metall, einschließlich Gadolinium (Gd), Europium

(Eu), Gallium (Ga), Mangan (Mn), Eisen (Fe), Yttrium (Y) sowie deren Isotope. Dabei ist es außerdem bevorzugt, wenn der Komplexbildner für Metalle ausgewählt ist aus der Gruppe bestehend aus: Tetraazacyclododecantetraessigsäure (DOTA), Diethylentriaminpentaessigsäure (DTPA), BOPTA, EOB-DTPA, DTPA-DMA, HP-DOBA, DTPA-BMEA, HIDA, DTDP, Porphyrine, Texaphyrine, TEKES, Fullerene, Kronenether.

Diese Maßnahme hat den Vorteil, dass ein Kontrastmittel für die Magnetresonanztomographie (MRT) bereitgestellt wird, mit dem Prostatazellen und Prostatakarzinomzellen einschließlich solche in den Lymphknoten, zuverlässig dargestellt werden können. Damit wird ein lang bestehendes Bedürfnis erfüllt, da derartige zur Darstellung von Prostatakarzinommetastasen geeignete Kontrastmittel bislang nicht existieren. Diese Kontrastmittel ermöglichen eine sehr hohe anatomische Auflösung. Dies gilt insbesondere für gadoliniumhaltige Kontrastmittel in Bezug auf T1-gewichtete Aufnahmen. Damit lassen sich Raumforderungen in kleinen Strukturen wie Lymphknoten lokalisieren. Eine derart hohe anatomische Auflösung ist durch Eisenkontrastmittel, welche über Suszeptibilitätsartefakte in T2-gewichteten Aufnahmen sichtbar werden, nicht möglich.

Bei dem Marker kann es sich ferner um einen solchen handeln, der mittels Computertomographie detektierbar ist und vorzugsweise eine Iodverbindung, einschließlich lopromid, Thyroxin, Triiodothyronin und Triiodobenzoesäure, aufweist.

Mit dieser Maßnahme wird ein solches Mittel bereitgestellt, mit dem Prostatazellen sowie Prostatakarzinomzellen einschließlich solche in den Lymphknoten, computertomographisch dargestellt werden können.

Darüber hinaus kann es sich bei dem Marker um einen solchen handeln, der mittels nuklearmedizinischer Verfahren detektierbar ist und vorzugsweise einen γ-Strahler wie Gadolinium-153 oder einen β-Strahler einschließlich Sr-89, Y-90, I-131, Er-169, Re-186 und Re-188, aufweist.

Mit dieser Maßnahme wird ein solches Mittel bereitgestellt, mit dem im Rahmen von nuklearmedizinischen Verfahren Prostatazellen bzw. Prostatakarzinomzellen einschließlich solche in den Lymphknoten dargestellt werden können.

Es versteht sich, dass das erfindungsgemäße Mittel nicht nur einen sondern mehrere Marker aufweisen kann. So ist es möglich, dass das erfindungsgemäße Mittel mehrere Moleküle des gleichen Markers aufweist, beispielsweise mehrere Gd-DOTA-Verbindungen. Eine solche Verbindung hat den Vorteil, dass sie in der Magnetresonanztomographie ein besonders starkes Signal liefert. Es ist jedoch auch möglich, dass das erfindungsgemäße Mittel mehrere verschiedene Marker aufweist. Dabei kann es sich um verschiedene Marker handeln, die mittels desselben bildgebenden Verfahrens detektierbar sind, oder aber um verschiedene Marker, die mittels unterschiedlicher bildgebender Verfahren detektierbar sind. Das erfindungsgemäße Mittel kann deshalb beispielsweise eine Gd-DOTA-Verbindung und eine FITC- und/oder Rhodamin-Verbindung aufweisen. Diese Maßnahme hat den Vorteil, dass das erfindungsgemäße Mittel sowohl in der Magnetresonanztomographie als auch der Fluoreszenzmikroskopie detektierbar ist und sich in beiden Verfahren Prostatazellen bzw. Prostatakarzinomzellen einschließlich solche in den Lymphknoten darstellen lassen.

Das für die Marker Erläuterte gilt entsprechend für den Linker. So kann das erfindungsgemäße Mittel mehrere identische oder unterschiedliche Linker aufweisen.

Linker und Marker können direkt miteinander kovalent verbunden sein. Marker und Ligand können jedoch auch über einen sogenannten "Linker" verbunden sein, der vorzugsweise 1, 2, 3, 4, 5, 6, 10, 15 bzw. beliebig viele Aminosäuren aufweist. Das Vorsehen eines solchen Aminosäurelinkers hat den Vorteil, dass sich der Abstand zwischen Marker und Ligand vergrößert, was sich positiv auf die Interaktion mit dem Rezeptor ausüben kann.

Besonders geeignete Linker enthalten Lysinreste, da diese eine freie Aminogruppe aufweisen, an die Marker bzw. Liganden über eine Peptidbindung angekoppelt werden können. Beispiele für geeignete Linker sind Folgende, wobei vereinbarungsgemäß auf der linken Seite der N-Terminus und auf der rechten Seite der C-Terminus steht: Lysin-Gycin-Lysin-Glycin, Lysin-Ahx-Lysin-Ahx (Ahx: Aminohexansäure), Lysin-ß-Ala-Lysin-ß-Ala, Glutaminsäure.

Dabei ist es bevorzugt, wenn das Mittel eine Verbindung aufweist, die ausgewählt ist aus der Gruppe bestehend aus:

### Sulforhodaminsulfonamido-Aminoundecanal (Verbindung 1)

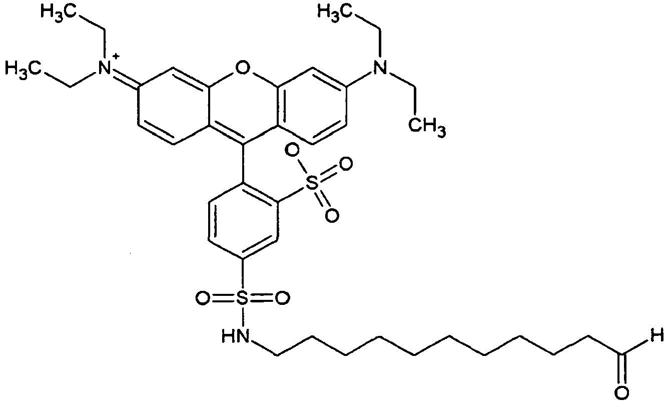

### Sulforhodaminsulfonamido-ßAla-Aminozimtaldehyd (Verbindung 2)

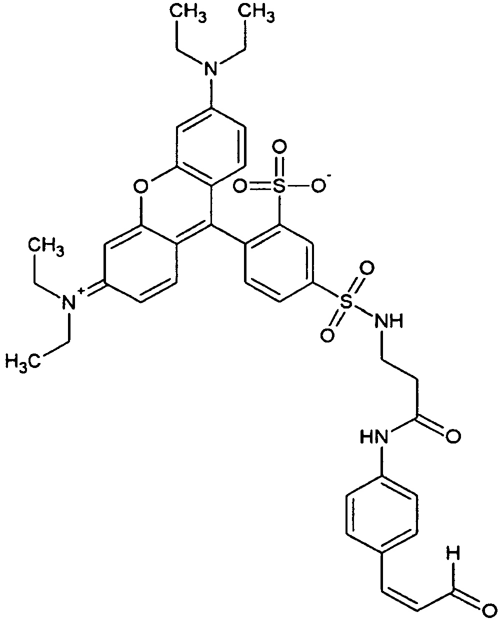

### Sulforhodaminsulfonamido-Aminobenzaldehyd (Verbindung 3)

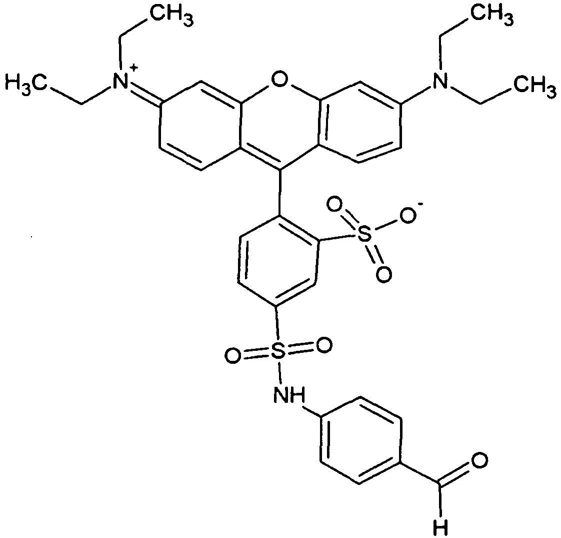

### Sulforhodaminsulfonamido-ß-Ala-Aminooctanal (Verbindung 4)

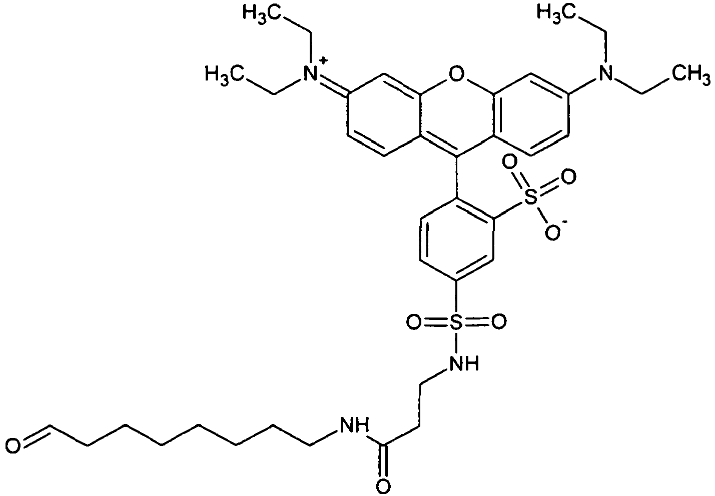

### Sulforhodaminsulfonamido-ßAla-Aminoundecanal (Verbindung 5)

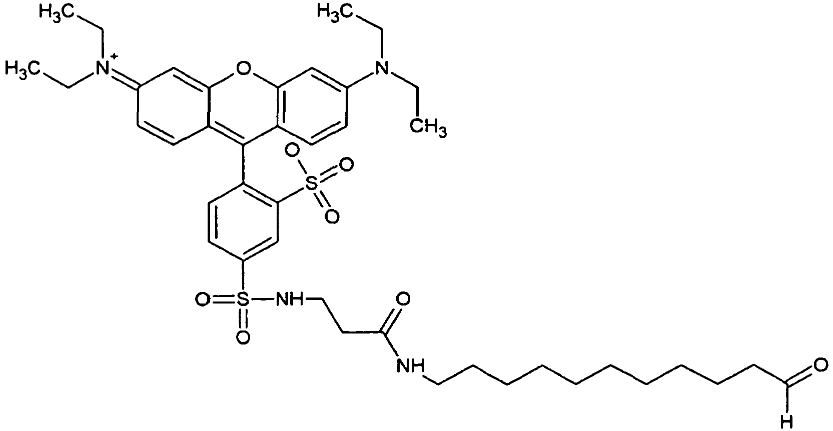

### FITC-ßAla-Aminoundecanal (Verbindung 6)

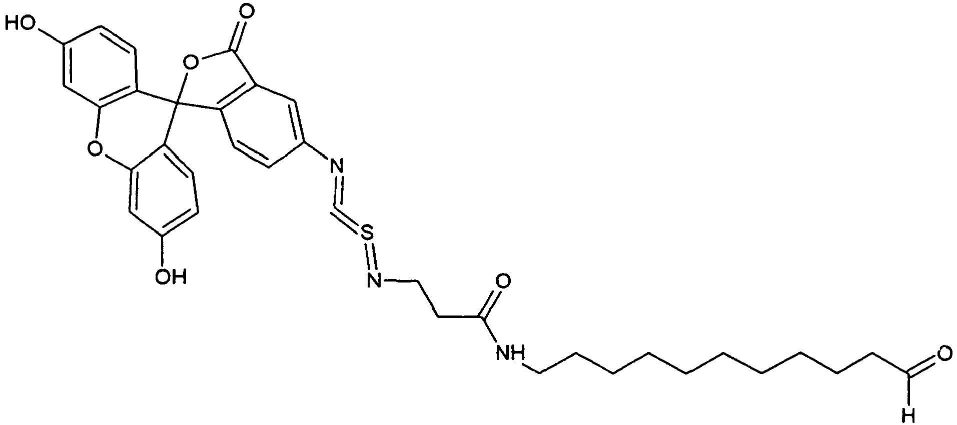

Die vorgenannten Mittel weisen die Fluoreszenzmarker Rhodamin bzw. Sulforhodaminsulfonamin und FITC auf und können deshalb in der Fluoreszenzmikroskopie detektiert werden. Die Verbindungen 2, 4, 5 und 6 weisen einen aus einer Aminosäure (Alanin) bestehenden Linker auf, über den der Ligand mit dem Marker verbunden ist.

### Lys(FITC)Aminobourgeonal (Verbindung 7)

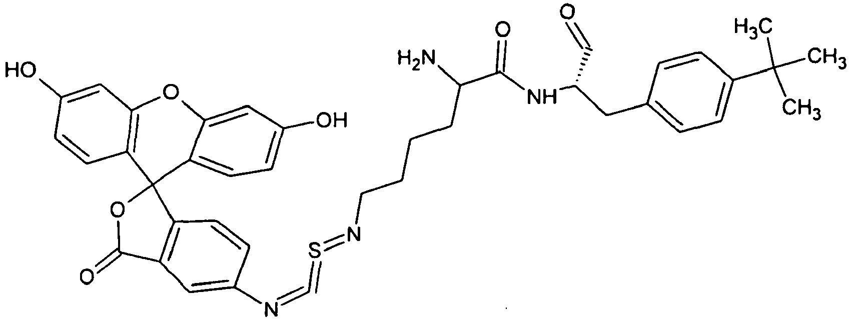

Die vorgenannten Mittel sind nach Erkenntnissen des Erfinders besonders geeignet.

Nach einer bevorzugten Weiterbildung weist das erfindungsgemäße Mittel eine Verbindung auf, die ausgewählt ist aus der Gruppe bestehend aus:

### Gd-DOTA-Aminoundecanal (Verbindung 8)

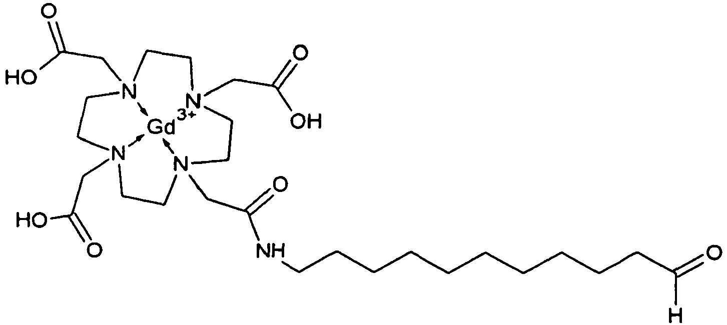

### Gd-DOTA-ßAla-Aminoundecanal (Verbindung 9)

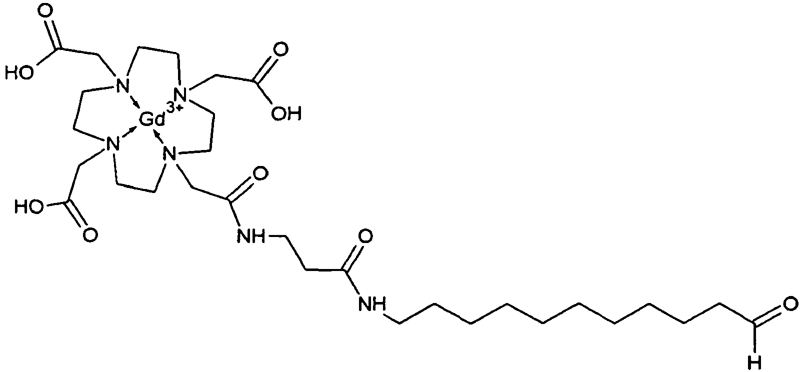

Mit dieser Maßnahme werden erfindungsgemäß solche Verbindungen bereitgestellt, die in der Magnetresonanztomographie eingesetzt werden können. In der Verbindung 8 ist der Ligand, das Undecanal bzw. Aminoundecanal direkt an den Marker Gd-DOTA gebunden. Bei der Verbindung 9 ist zwischen dem Liganden und dem Marker ein Linker in Form einer Aminosäure (β-Alanin) angeordnet.

Nach Erkenntnissen des Erfinders sind die vorgenannten Mittel besonders geeignet, um in der Magnetresonanztomographie Prostatazellen bzw. Prostatakarzinomzellen einschließlich solcher in den Lymphknoten darzustellen.

Weiter ist es bevorzugt, wenn das Mittel eine Verbindung aufweist, die ausgewählt ist aus der Gruppe bestehend aus:

### Gd-DOTA-Lys(FITC)-Aminoundecanal (Verbindung 10)

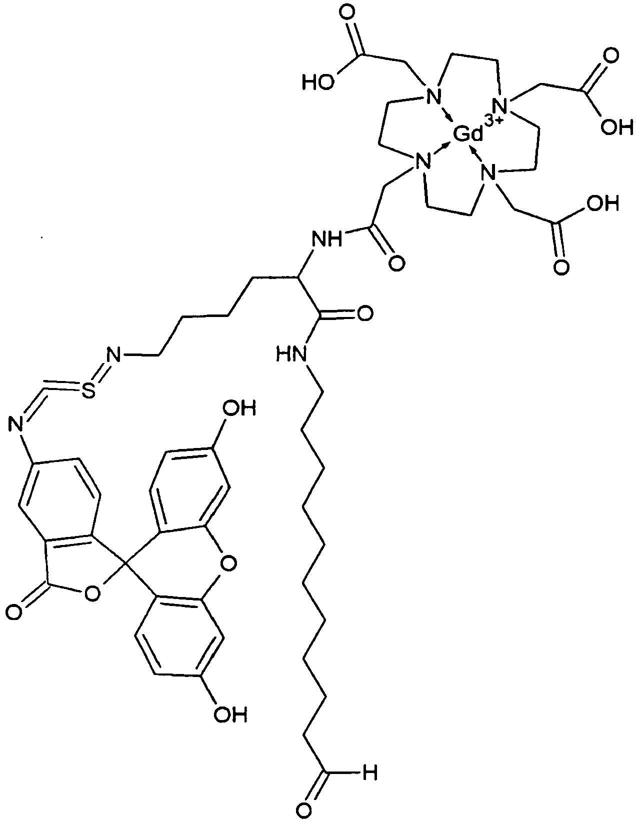

### Gd-DOTA-ßAla-Lys(FITC)-Aminoundecanal (Verbindung 11)

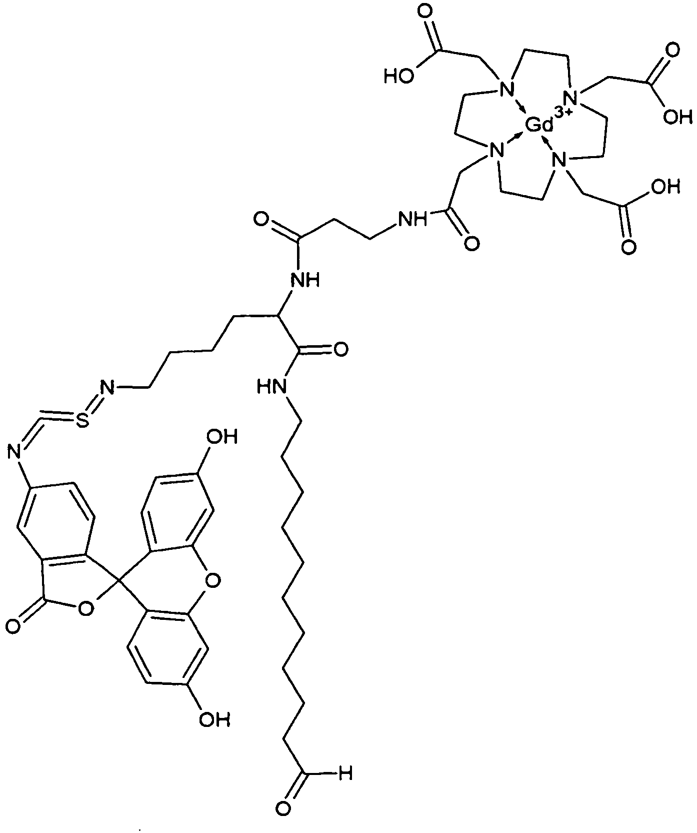

### FITC-ßAla-Lys(Gd-DOTA)-Aminoundecanal (Verbindung 12)

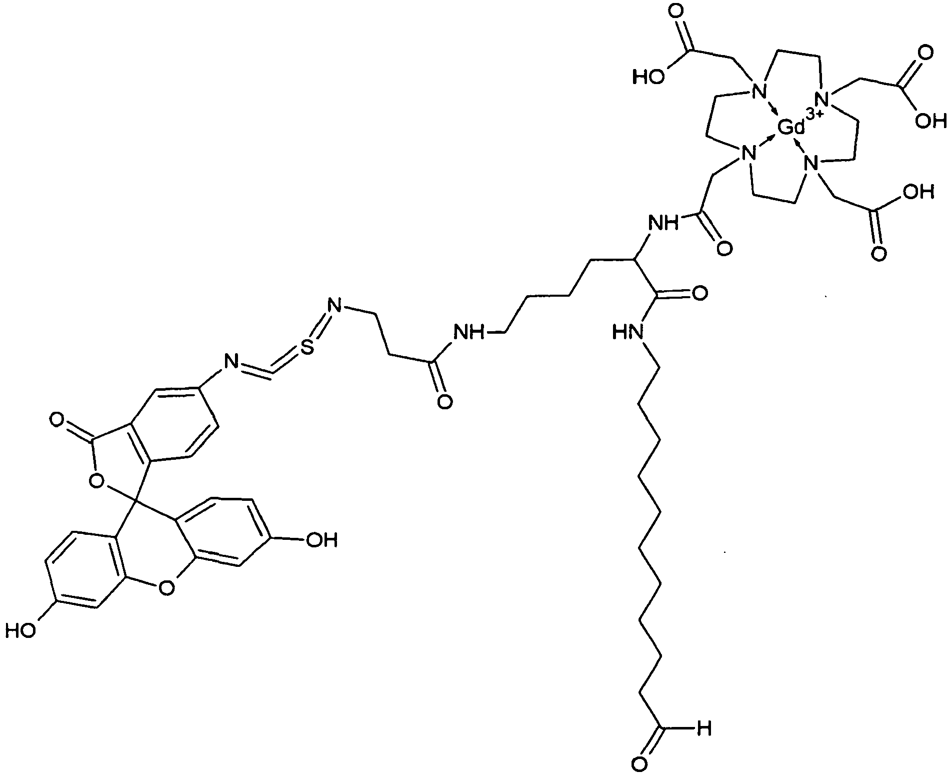

### Gd-DOTA-ßAla Lys(FITC)-IPLVVPL-Aminoundecanal (Verbindung 13)

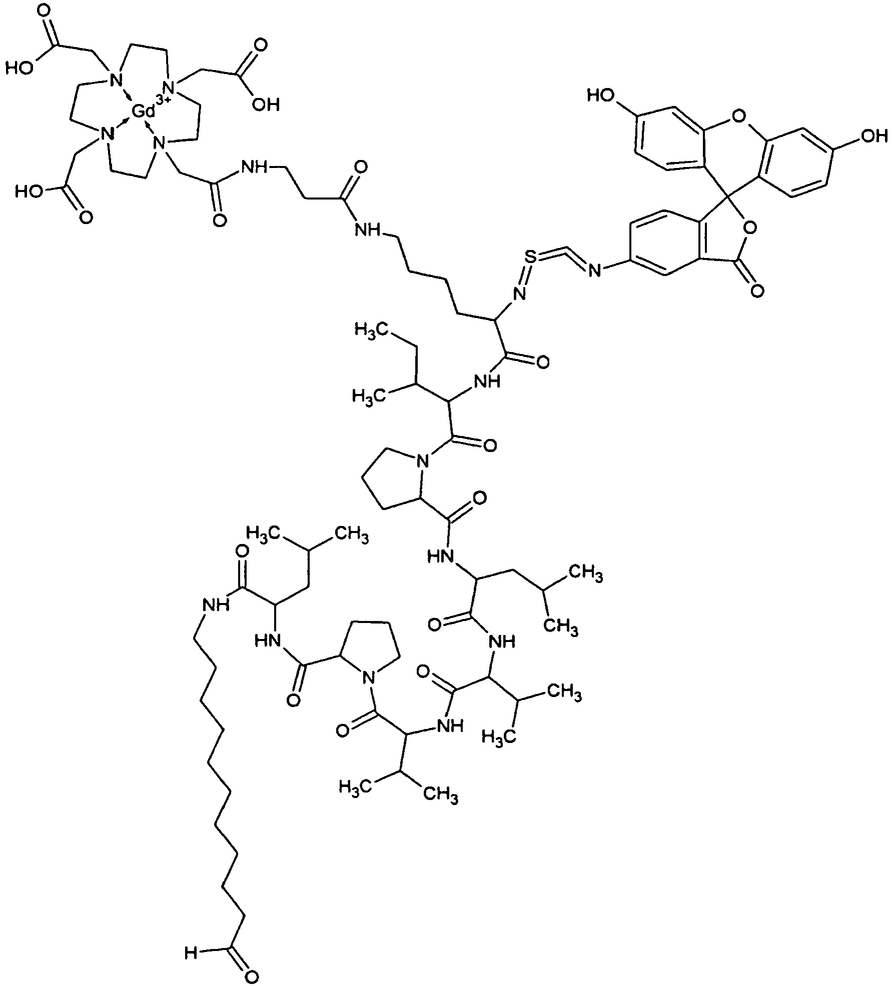

### Gd-DOTA(4)-Lys(4)-Lys(2)-Lys(FITC)-Aminoundecanal (Verbindung 14)

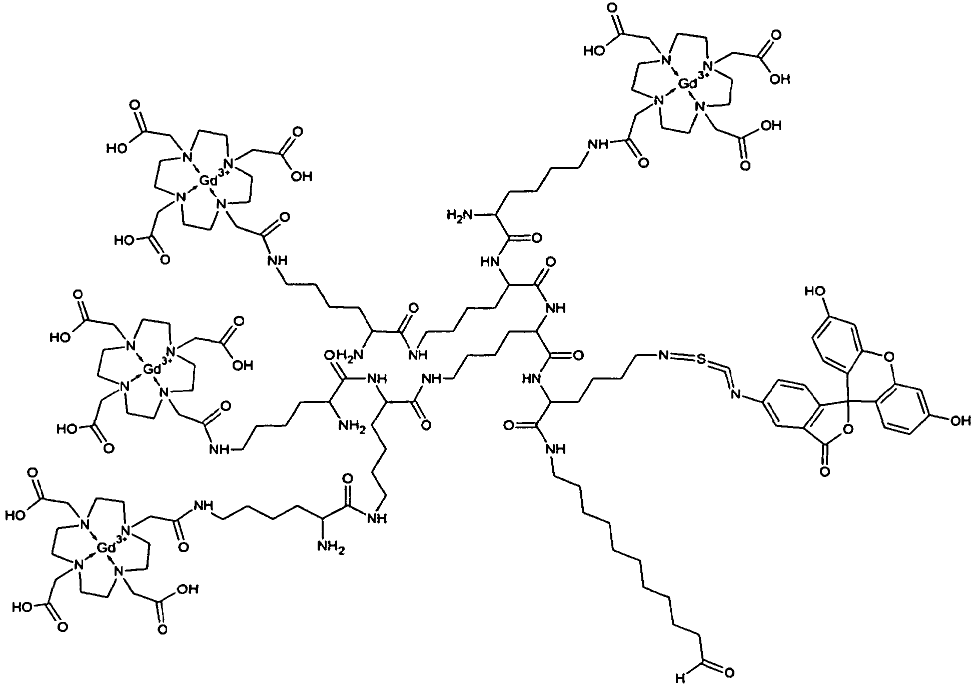

Mit dieser Maßnahme werden solche erfindungsgemäßen Mittel bereitgestellt, die sowohl in der Magnetresonanztomographie als auch der Fluoreszenzmikroskopie einsetzbar sind. Die Verbindungen weisen sowohl einen in der Magnetresonanztomographie detektierbaren Marker auf, nämlich Gd-DOTA, als auch einen in der Fluoreszenzmikroskopie detektierbaren Marker, nämlich FITC. In der Verbindung 10 ist der Ligand über ein Lysinrest an den in der Magnetresonanz detektierbaren Marker Gd-DOTA gebunden. An die Seitenkette des Lysinrestes ist der Fluoreszenzmarker FITC gekoppelt.

Bei der Verbindung 11 ist der Ligand über einen aus Alanin und Lysin bestehenden Aminosäurelinker mit dem MRT-Marker Gd-DOTA verbunden, wobei an den Lysinrest ein FITC-Marker gekoppelt ist, der in der Fluoreszenzmikroskopie sichtbar ist.

Die Verbindung 12 unterscheidet sich von der Verbindung 10 darin, dass die Fluoreszenz- bzw. MRT-Marker in ihrer Anordnung vertauscht sind.

Die Verbindung 13 ist dadurch gekennzeichnet, dass der Ligand mit dem MRT-Marker über einen neun Aminosäuren aufweisenden Linker verbunden ist, wobei an dem Lysinrest über die Seitenkette der Fluoreszenzmarker FITC angekoppelt ist.

Die Verbindung 14 ist dadurch gekennzeichnet, dass sie vier Gd-DOTA-Marker und einen FITC-Marker aufweist, die miteinander über einen aus sieben Aminosäuren bestehenden Linker verbunden sind. Diese Verbindung liefert in der Magnetresonanztomographie ein besonders starkes Signal.

Es versteht sich, dass die vorstehend genannten Verbindungen lediglich Beispiele sind. Es ist in das Belieben des Fachmannes gestellt, welchen Liganden, welchen Marker und welche Anzahl hiervon miteinander auf geeignete Art und Weise verbunden und in Form des erfindungsgemäßen Mittels bereitgestellt werden.

Bei dem erfindungsgemäßen Mittel handelt es sich vorzugsweise um ein Diagnostikum zum Nachweis von Prostatakarzinomen.

Wie der Erfinder in Versuchen *in vivo* an einem Prostatakarzinom auf der Nacktmaus feststellen konnte, konnte mit dem erfindungsgemäßen Mittel das Prostatakarzinomgewebe gut angefärbt werden, während das gesunde Prostatagewebe der Maus keine nennenswerte Färbung zeigte. Die Unterscheidung von Prostatakarzinomgewebe und gesundem Prostata- sowie Nicht-Prostatagewebe ist deshalb mittels des erfindungsgemäßen Mittels gut möglich und diagnostisch nutzbar.

Besonders gut lassen sich mit dem erfindungsgemäßen Mittel Prostatakarzinommetastasen in Lymphknoten und/oder Knochen darstellen. Lymphozyten weisen an ihrer Oberfläche keine Maiglöckchenduftrezeptoren auf und können deshalb das erfindungsgemäße Mittel nicht binden bzw. aufnehmen. Mit dem erfindungsgemäßen Mittel ist deshalb erstmals eine Positivdarstellung von Prostatakarzinomzellen in den Lymphknoten möglich.

Bei dem erfindungsgemäßen Mittel kann es sich auch um ein Therapeutikum handeln.

Durch die hohe Selektivität und Spezifität können mit dem erfindungsgemäßen Mittel Arzneimittel den Prostatazellen bzw. Prostatakarzinomzellen zugeführt werden. Diese Maßnahme hat den Vorteil, dass die Wirkung ausschließlich in den Prostatazellen bzw. Prostatakarzinomzellen eintritt und anderes Gewebe und damit der Organismus verschont bleiben. Ein solches Therapeutikum ist besonders nebenwirkungsarm.

Vorzugsweise weist das als Therapeutikum ausgestaltete erfindungsgemäße Mittel ein Zytostatikum auf, bei dem es sich um ein Alkylanz, Platinanalogon, Interkalanz, Antibiotikum, Mitosehemmer, Texan, Topoisomerasehemmer und Antimetabolit handeln kann.

Mit dieser Maßnahme wird ein solches Therapeutikum bereitgestellt, mit dem zielgerichtet und selektiv ausschließlich Prostatakarzinomzellen in ihrer Teilung inhibiert und damit abgetötet werden können. Zellen anderer Gewebe bleiben hiervon weitgehend unbeeinträchtigt.

Es versteht sich, dass das erfindungsgemäße Mittel sowohl als Diagnostikum als auch als Therapeutikum ausgestaltet sein kann. So kann das Mittel sowohl einen mittels bildgebender Verfahren detektierbaren Marker und gleichzeitig ein Zytostatikum aufweisen. Auf diese Art und Weise kann der Therapieverlauf visualisiert werden.

Die Merkmale, Eigenschaften und Vorteile des erfindungsgemäßen Mittels gelten entsprechend für das erfindungsgemäße Verfahren.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nun anhand von Ausführungsbeispielen näher erläutert, aus denen sich weitere Eigenschaften, Merkmale und Vorteile ergeben.

Dabei wird Bezug genommen auf die beigefügten Figuren, in denen Folgendes zu sehen ist:
- Fig. 1A: zeigt Aufnahmen aus der konfokalen Laserscanningmikroskopie (CLSM) von sieben humanen Tumorzelllinien (PC-3/LNCaP - Prostatakarzinom; Colo 205 - Kolonkarzinom; U373 - Gliom; U2OS - Osteosarkom; HeLa - Zervixkarzinom; Jurkat - Lymphom) und gesunden humanen epithelialen Prostatazellen (CC2555, Lonza) nach 20-minütiger Inkubation mit Sulforhodaminsulfonamido-Aminoundecanal (Verbindung 1).
- Fig. 1B: zeigt CLSM-Aufnahmen von Prostatakarzinomzellen (LNCaP) und Kolonkarzinomzellen (Colo205) 20 Minuten nach der Inkubation mit Sulforhodaminsulfonamido-βAla-Zimtaldehyd (Verbindung 2).
- Fig. 2A: zeigt CLSM-Aufnahmen von Prostatakarzinomzellen (PC-3) und Osteosarkomzellen (U2OS) 20 Minuten nach der Inkubation mit Gd-DOTA-Lys(FITC)-Aminoundecanal (Verbindung 10).
- Fig. 2B: zeigt Aufnahmen aus der fluoreszenzaktivierten Zellsortierung (FACS) von sieben Tumorzelllinien (PC-3/LNCaP - Prostatakarzinom; Colo205 - Kolonkarzinom; U373 - Gliom; U20S - Osteosarkom; HeLa - Zervixkarzinom; Jurkat - Lymphom) und gesunden humanen epithelialen Prostatazellen (CC2555, Lonza) nach Inkubation mit Gd-DOTA-Lys(FITC)-Aminoundecanal (Verbindung 10) (dreifache Ausführung, Standardabweichung vom Mittelwert).
- Fig. 2C: zeigt Aufnahmen einer FACS-Analyse von verschiedenen Karzinomzellen 20 Minuten nach der Inkubation mit Sulforhodaminsulfonamido-β-Ala-Aminooctanal (Verbindung 4) (dreifache Ausführung, Standardabweichung vom Mittelwert).
- Fig.2D: zeigt LSM-Aufnahmen von PC-3-Prostatakarzinomzellen und U373-Gliomzellen 48 Stunden nach Inkubation mit Lys(FITC)-Aminobourgeonal (Verbindung 7).
- Fig. 3A: zeigt magnetresonanztomographische Aufnahmen von seitlich angeschnittenen Eppendorfröhrchen mit LNCaP-Zellen nach 20-minütiger Inkubation mit Gd-DOTA allein (DOTAREM) (links) und dem Gd-DOTA-Lys(FITC)-Aminoundecanal (Verbindung 10) (rechts).
- Fig. 3B und C: zeigen den Einfluss der Kopplung von Undecanal an Gd-DOTA.
- Fig. 3D: zeigt magnetresonanztomographische Aufnahmen eines humanen PC3-Prostatakarzinoms in der rechten unteren Flanke einer Nacktmaus (Pfeil) (10 Tage nach der Implantation) vor und 90 Minuten nach intravenöser Gabe von Gd-DOTA-Lys(FITC)-Aminoundecanal (Verbindung 10).
- Fig. 4: zeigt CLSM-Aufnahmen von 5 µm Gefrierschnitten von Nacktmausorganen und einem humanen Prostatakarzinom (PC3) 90 Minuten nach intraperitonealer Injektion von Sulforhodaminsulfonamido-Amidoundecanal (Verbindung 1) (Überlagerung Transmission und Rhodamin-Kanal jeweils links, nur Rhodamin-Kanal jeweils rechts).
- Fig. 5A: zeigt fluoreszenzmikroskopische Aufnahmen von 5 µm Gefrierschnitten eines Prostatakarzinoms (PC3) 20 und 180 Minuten nach intraperitonealer Injektion von Sulforhodaminsulfonamido-Aminoundecanal (Verbindung 1).
- Fig. 5B: zeigt Aufnahmen eines "Abklatschpräparates" ("Touch Print") des PC3-Prostatakarzinoms 20 Minuten nach intraperitonealer Injektion von Sulforhodaminsulfonamido-Aminoundecanal (Verbindung 1).

### Ausführungsbeispiele

### 1. Material und Methoden

### 1.1 Synthese von Sulforhodaminsulfamido-Aminoundecanal (Verbindung 1)

Weinreb-AM-Harz (NovaBiochem) [0,25mmol (0,28g)] wurde in 10 ml Dimethylformamid (DMF) suspendiert und 10 Minuten quellen gelassen. Dann wurde die Fmoc-Gruppe durch Zugabe von 25% Piperidin in DMF (3 Minuten, 6 Minuten, 2 Minuten) entfernt. Nach mehrfachem Waschen mit DMF wurde die dreifache Menge (0,75 mmol) Fmoc-Aminoundecansäure mit Hilfe von 0,75 mmol TBTU [O-(Benzotriazol-1-yl)-N,N,N',N'-tetramethyluronium tetrafluoroborat] in Gegenwart von 6 Äquivalenten Diisopropylethylamin (DIEA) innerhalb von 1h bei Raumtemperatur auf das Harz gekoppelt.

Nach wiederholter Fmoc-Abspaltung wurden 1,5 Äquivalente Sulforhodamin-B- Säurechlorid und 1,5 Äquivalente DIEA gelöst und in einem 1:1 Gemisch aus DMF/Dichlormethan zum Ansatz gegeben und 6h bei Raumtemperatur geschüttelt.

Nach Auswaschen des überschüssigen Farbstoffes suspendierte man das Peptidharz in absolutem Tetrahydrofuran (THF), gab 2,5 ml einer 1molaren Lithium-aluminiumhydridlösung unter Eiskühlung hinzu und rührte das Ganze 45 Minuten.

Nach Hydrolyse durch vorsichtige Zugabe von 2N Zitronensäure trennte man vom Harz ab und verdünnte mit Dichlormethan (DCM). Die DCM-Lösung wurd mit 2N Zitronensäure und gesättigter NaCl-Lösung gewaschen und mit Na₂SO₄ getrocknet. Das Produkt wurde über eine Flüssigkeitschromatographie an einer Kieselgelsäule mit Ethylether/Petrolether gereinigt.

Das Produkt wird durch Elektrosprayionisierungsmassenspektrometrie (ESI-MS) verifiziert.

### 1.2 Synthese von Sulforhodaminsulfonamido-βAla-Aminozimtaldehyd (Verbindung 2)

Die Synthese erfolgte wie unter 1.1. Anstelle Fmoc-Aminoundecansäure wurde Fmoc-Aminozimtsäure verwendet.

### 1.3 Synthese von Sulforhodaminsulfonamido-β-Ala-Aminooctanal (Verbindung 4)

Die Synthese erfolgte wie unter 1.1. Anstelle Fmoc-Aminoundecansäure wurde Fmoc-Aminooctansäure verwendet.

### 1.4 Synthese von Lys(FITC)-Aminobourgeonal (Verbindung 7)

Die Synthese erfolgte wie unter 1.1 und 1.5. Anstelle Fmoc-Aminoundecansäure wurde Fmoc-p-tBu-Phe-OH verwendet.

### 1.5 Synthese von DOTA-Lys(FITC)-Aminoundecanal und dessen Gadoliniumkomplex (Verbindung 10)

Weinreb-AM-Harz (NovaBiochem) [0.25 mM (0.28 g)] wurde mit 25% Piperidin/DMF (3 Minuten, 6 Minuten, 2 Minuten) von der Fmoc-Gruppe befreit. Durch Kopplung von 3 Äquivalenten Fmoc-Aminoundecansäure mit TBTU [O-(Benzotriazol-1-yl)-N,N,N',N'-tetramethyluronium tetrafluoroborat] und 6 Äquivalenten Diisopropylethylamin (DIEH) erhielt man Fmoc-AM-Weinreb-Harz.

Nach erfolgter Abspaltung der Fmoc-Gruppe koppelte man unter Verwendung von TBTU Fmoc-Lys (Mmt)-OH an das Harz (Mmt: Methoxytrityl). Wiederum nach Abspaltung der Fmoc-Gruppe erfolgte die Kopplung von DOTA (1.4.7.10-Tetraazazyclodododecan-1.4.7 tri-tert.butylessigester-10-Essigsäure) an das Lysin in Gegenwart von TBTU und DIEA.

Nach Abspaltung der Mmt-Schutzgruppe, durch mehrfaches Übergiessen des Harzes mit 1% Trifluoressigsäure-Lösung in Dichlormethan innerhalb einer Stunde bei Raumtemperatur, und Neutralisation des Harzes durch Zugabe von DIEA wurde FITC (Fluorescein Isothiocyanat) in DMSO gelöst und in Gegenwart von Diisopropylethylamin über Nacht auf das Harz gekoppelt.

Nach mehrfachem Auswaschen des überschüssigen Farbstoffes und Trocknen des Harzes wurde das Harz in THF suspendiert, auf 5°C gekühlt und mit 2.5 ml 1 M Lithiumaluminiumhydrid in THF versetzt und 45 min bei Raumtemperatur gerührt.

Durch vorsichtige Zugabe von 2 N Zitronensäure wurde der Ansatz hydrolysiert. Dann trennte man von polymeren Bestandteilen und versetzte mit Dichlormethan. Nach mehrmaligem Waschen mit verdünnter Zitronensäure und NaCl-Lösung trocknete man über Natriumsulfat.

Nach Abziehen des Lösungsmittels wurden die verbliebenen tert-Butylestergruppen durch Übergiessen mit Trifluoressigsäure und halbstündigem Rühren bei Raumtemperatur entfernt. Nach Abziehen der Trifluoressigsäure wurde in 10 % Essigsäure aufgenommen und lyophilisiert.

Die so erhaltene Substanz wurde durch Zugabe von 1 Äquivalent Gadolinium-III-chlorid-hexahydrat in Wasser mit verdünnter Natronlauge auf pH 5.6 gebracht und bei 50°C 5 h gerührt. Nach Ansäuern mit verdünnter Essigsäure wurde wiederum lyophilisiert.

Das Produkt wurde durch Elektrosprayionisierungsmassenspektrometrie (ESI-MS) verifiziert.

### 1.6 Elektrosprayionisierungsmassenspektrometrie (ESI-MS)

Die Konjugate wurden durch ESI-MS auf einem Esquire3000+ Ion Trap-Massenspektrometer (Bruker-Daltonics, Bremen, Deutschland) analysiert. Die Konjugate wurden in 40 % ACN, 0,1 % Ameisensäure (Sigma-Aldrich) in Wasser (v/v/v) (20 pmol/µl) aufgenommen und unter Verwendung einer Spritzenpumpe konstant infundiert (5 µl/min Flussrate). Die Massenspektren wurden im positiven Ionenmodus gewonnen. Die Temperatur des Trockengases (6 1/min) wurde auf 325°C, der Vernebler auf 20.0 psi und die Elektrosprayspannung auf -3700 V eingestellt.

### 1.7 Konfokale Laserscanningmikroskopie (CLSM)

Die Zellen wurden bis zu 80 % Konfluenz bei 37°C, 5 % CO₂ (vol/vol) in Platten mit 4 Vertiefungen (NUNC, Wiesbaden, Deutschland) mit etwa 300.000 Zellen pro Vertiefung wachsen gelassen.

Humane Gliomzellen U373, humane Prostatakarzinomzellen PC3 und LNCaP, humane Jurkat-Lymphomzellen und humane Kolonkarzinomzellen Colo205 wurden in RPMI1640 Ready Mix Medium wachsen gelassen, das L-Glutamin und 10 %-iges fötales Rinderserum (FBS)-Gold enthielt (PAA Laboratories, Pasching, Österreich). Die humanen Osteosarkomzellen U2OS und humanen Zervix-Karzinomzellen HeLa wurden in Dulbeccos modifiziertem Eagle's Medium mit hoher Glucose und GlutaMax™ sowie Pyrovat und 10 % FBS-Gold wachsen gelassen. Gesunde humane Prostatazellen (CC2555, Lonza, Verviers, Belgien) wurden in Prostatapithelzellwachstumsmedium (PrEGM, Lonza, Verviers, Belgien) wachsen gelassen.

Die Verbindungen 1, 2 und 4 wurden in 1 mg/ml reinem Ethanol (Riedel de Haen, Sigma-Aldrich, Taufkirchen, Deutschland) gelöst und dann in dem für die Zellen geeigneten Medium auf 10 µg/ml Konjugat und 1 % Ethanol verdünnt. Die Zellen wurden bei 37°C in einer Atmosphäre mit 5 % CO₂ für 20 Minuten mit den Verbindungen inkubiert. Anschließend wurden die Zellen dreimal mit Medium gespült und erneut in Medium inkubiert. Dieser Versuch wurde auch mit freiem Sulforhodamin B in entsprechender Menge durchgeführt.

Um Phosphatidylserin darstellen zu können (Apoptosetest) wurde Annexin-Fluos (Roche Diagnostics, Mannheim, Germany) hinzugegeben. Dies wurde sowohl nach 20-minütiger als auch nach 2-tägiger Inkubation mit den Verbindungen 1, 2 und 4 durchgeführt.

Die Verbindung 10 wurde im Kulturmedium gelöst (260 µM). Lys(FITC)-Aminobourgeonal (Verbindung 7) wurde in Kulturmedium mit 1% Ethanol gelöst (260 µM). Die Zellen wurden für 20 Minuten (Verbindung 10) und für 2 Tage (Verbindungen 7 und 10) inkubiert. Dieser Versuch wurde auch mit freiem FITC durchgeführt. Nach Inkubation wurden die Zellen dreimal mit Medium gespült und erneut in Medium inkubiert. Propidium Iodide (PI) (Molecular Probes, Eugene, OR, USA) wurde hinzugegeben um tote Zellen zu färben.

Die konfokale Laserscanningmikroskopie wurde auf einem invertierten LSM510-Laserscanningmikroskop (Carl Zeiss) (Objektive: LD Achroplan 40x0,6, Plan Neofluar 20x0,50, 40x0,75) durchgeführt. Für die Rhodaminfluoreszenzanregung wurde der 543 nm-Strahl eines Heliumneonlasers mit geeigneten Strahlenteilern und Grenzfiltern verwendet. Sämtliche Messungen erfolgten zumindest dreimal an lebenden, nicht fixierten Zellen.

### 1.8 Schnitte von Mausorganen und Abklatschpräparate ("Touch Prints")

PC3- und LNCaP-Prostatatumorzellen wurden bis zu einer 80 %-igen Konfluenz wachsen gelassen, mit Accutase™ (PAA Laboratories, Pasching, Österreich) abgelöst und dann geerntet. Die Zellen wurden dreimal mit PBS gewaschen und dann in eine 30 g Micro-Fine™ Insulinspritze (Becton Dickinson, Franklin Lakes, NJ, USA) aufgenommen. Etwa 5x10⁵ Zellen pro Maus wurden in die rechte untere Flanke einer männlichen CD1 Nu/Nu-Maus (Charles River WIGA, Sulzfeld, Deutschland) injiziert. Das Tumorwachstum wurde beobachtet und nach 10 bis 14 Tagen wurden Mäuse, die Tumoren mit 2-3 mm im Durchmesser trugen, mit dem Konjugat behandelt. 1,5 mg der Verbindung 1 wurden in 300 µl PBS gelöst, welcher 20 % Ethanol enthielt, und intraperitoneal verabreicht. Nach 90 Minuten der Zirkulation wurden die Mäuse getötet und anschließend wurden die Tumoren und Organe entfernt, in Jung-Gefriermedium (Leica Instruments, Nussloch, Deutschland) eingebettet und in flüssigem Stickstoff eingefroren. Gefrierschnitte (5 µm Dicke) wurden hergestellt (LC3000 Cryotom, Leica Biosystems GmbH, Nussloch, Deutschland) und die Rhodaminfärbung wurde durch konfokales Laserscanning analysiert.

Um den In- und Efflux der Rhodamin-konjugierten Verbindungen 1 zu evaluieren, wurde das Experiment mit Zirkulationszeiten von 20 Minuten und 180 Minuten wiederholt.

Für die Abklatschpräparate wurde der Tumor einer Maus, die mit der Rhodamin enthaltenden Verbindung 1 (1,5 mg intraperitoneal für 20 Minuten) behandelt wurde, entfernt. Der Tumor wurde angeschnitten und die Zellen ohne Fixierung auf einem Objektträger ausgestrichen. Die Tumorzellen wurden sofort mittels CLSM (ohne Fixierung) analysiert.

Die Experimente wurden jeweils zweimal wiederholt.

### 1.9 Magnetresonanztomographiemessungen

Die magnetresonanztomographischen (MRT) Messungen erfolgten unter Verwendung eines klinischen 3-Tesla Siemens-Ganzkörper-MRT (Magnetom TRIO; Siemens, Erlangen, Deutschland) mit den Mäusen in Bauchlage in einer zirkular polarisierten Standard-Handgelenksspule. Das MRT-Protokoll bestand aus nativen und kontrastmittelangehobenen T1-gewichteten axialen Aufnahmen: Schichtdicke: 2 mm, Aufnahmefeld: 60 mm / 87,5 %, Voxel-Grösse: 0,2x0,2x2 mm, Repetitionszeit (TR): 800 ms, Echozeit (TE): 9,1 ms, Flip-Winkel: 90°, Akquisitionen: 2, Anzahl der Schichten: 25, Distanzfaktor: 0, Aufnahmezeit 06:02 min.

Die DOTA-enthaltende Verbindung 9 wurde drei narkotisierten Mäusen intravenös über die Schwanzvene verabreicht (3 mg in 200 µl PBS).

### 1.10 MR-Relaxometrie

Für die MR-Relaxometrie wurden alle Tumorzelllinien in 75 cm² Kulturflaschen (Corning Costar, Bodenheim, Deutschland) wachsen gelassen (70 % Konfluenz). Accutase™ (PAA Laboratories, Pasching, Österreich) wurde hinzugegeben, um die Ablösung der Zellen zu erreichen, die geerntet und anschließend in Eppendorfröhrchen aliquotiert wurden (6 x 10⁶ Zellen pro Röhrchen). Die Zellen in den Röhrchen wurden mit DOTA-enthaltenden Verbindungen inkubiert (130 µM). Nach einer 20-minütigen Inkubation bei 37°C in einer Atmosphäre mit 5 % CO₂ wurden die Zellen dreimal in PBS gewaschen und bei 800 rpm für 5 Minuten zentrifugiert.

Die In-vitro-MRT erfolgte mit einem 3 Tesla-Ganzkörper-MRT-System (Magnetom TRIO, zirkular polarisierte Standard-Handgelenksspule, Siemens, Erlangen, Deutschland).

Sagittale T1-gewichtete MR-Aufnahmen wurden erhalten, indem die folgende Spin-Echo-Sequenz verwendet wurde:

Repetitionszeit (TR): 200 ms, Echozeit (TE): 7,4 ms, Flip-Winkel: 90°, Mittelungen: 1, Konkatenationen: 2, Messungen: 1, Anzahl der Schichten: 19, Distanzfaktor: 30 %, Schichtdicke: 3 mm, Aufnahmefeld: 180 mm / 100 %, Auflösung: 256 / 100 %, Voxel-Grösse: 0,7 x 0,7 x 3,0 mm, Aufnahmezeit: 1:48 min.

Von den Signalintensitäten wurde die T1-Relaxionszeiten evaluiert, die durch multiple Spin-Echo-Sequenz-Messungen erhalten wurden:

Repetitionszeit (TR): 20-8000 ms (50 verschiedene TR-Werte), Echozeit (TE): 6,4 ms, Flip-Winkel: 90°, Mittelungen: 1, Messungen: 1, Anzahl der Schichten: 1, Schichtdicke: 1 mm, Aufnahmefeld: 120 mm / 87,5 %, Auflösung: 128 /100 %, Voxel-Grösse: 0,9 x 0,9 x 1 mm.

Die Analysen und Berechnungen erfolgten unter Verwendung eines Matlab-Programms (Math Works, Natick, MA, USA). Die T1-Werte wurden durch ein Drei-Parameter-Anpassungsverfahren approximiert. Sämtliche Signalkurven wurden geprüft und als monoexponential bestimmt. Die Untersuchungen erfolgten in Dreifachansätzen.

### 1.11 Durchflusszytometrie

Für die fluoreszenzaktivierte Zellsortierung (FACS) wurden die Zellen in 75 cm² Kulturflaschen (Corning Costar, Bodenheim, Deutschland) wachsen gelassen (80 % Konfluenz), und zwar unter den gleichen Bedingungen, die für die konfokale Laser-Scanning-Mikroskopie beschrieben wurden. Accutase™ (PAA Laboratories, Pasching, Österreich) wurde hinzugegeben, um die Ablösung der Zellen zu erreichen, die geerntet und anschließend in Eppendorfröhrchen (Eppendorf, Hamburg, Deutschland) aliquotiert wurden (1x10⁶ Zellen pro Röhrchen). Die Zellen wurden für 20 Minuten mit den Verbindungen 1, 2 und 4 (10 µg/ml im entsprechenden Medium mit 1 % Ethanol) und der Verbindung 10 (130 µM im entsprechenden Medium) inkubiert.

Danach wurden die Zellen dreimal in PBS gewaschen und bei 800 rpm für 5 Minuten zentrifugiert. Anschließend wurden 300 µl FACS-Puffer (D-PBS mit 1 % Paraformaldehyd) hinzugegeben. Die Proben wurden sofort gemessen. Etwa 20.000 Ereignisse wurden pro Probe aufgenommen. Die Fluoreszenzanregung erfolgte mit einem Argonlaser (488 nm). Die Fluoreszenz wurde unter Verwendung eines 580-610 nm- und eines 560-680 nm-Bandpassfilters detektiert. Sämtliche Untersuchungen erfolgten in Dreifachansätzen.

Die Werte der mittleren Rhodamin- und Fluoresceinisothiocyanatfluoreszenz der Proben wurden unter Verwendung der WinMDI-Software (Joseph Trotter, Scripps Research Institute, San Diego, CA, USA) ermittelt und anschließend statistisch evaluiert.

### 2. Ergebnisse

### 2.1 Selektive Färbung von Prostatazellen in vitro

Sieben humane Tumorzelllinien und gesunde humane epitheliale Prostatazellen (CC2555) wurden 20 Minuten mit Sulforhodaminsulfonamido-Aminoundecanal (Verbindung 1) inkubiert und mittels konfokaler Laserscanningmikroskopie (CLSM) analysiert.

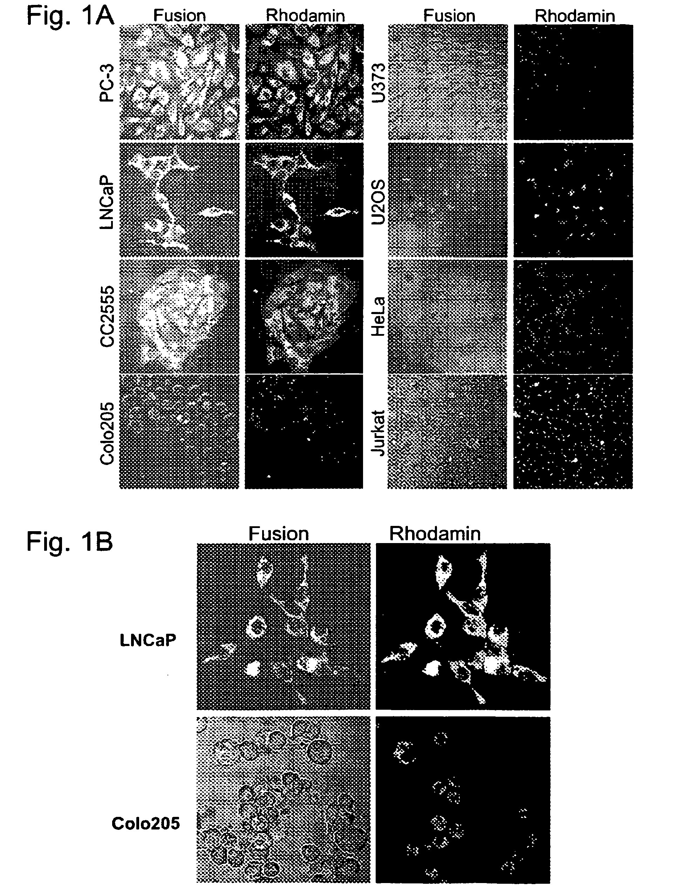

Das Ergebnis ist in Fig. 1A dargestellt. Dabei zeigt sich, dass die Prostatakarzinomzellen (PC-3, LNCaP) und die gesunden Prostatazellen (CC2555) die erfindungsgemäße Verbindung in das Zytoplasma aufnehmen, die Zellkerne bleiben ausgespart. Alle anderen Zellen hingegen nehmen die erfindungsgemäße Verbindung nicht auf. Rhodamin ohne Undecanal allein wird von sämtlichen Zelltypen nicht aufgenommen (nicht gezeigt).

In einem weiteren Ansatz wurden sowohl Prostatakarzinomzellen (LNCaP) als auch Kolonkarzinomzellen (Colo205) 20 Minuten mit Sulforhodaminsulfonamido-βAla-Zimtaldehyd (Verbindung 2) inkubiert und mittels CLSM analysiert.

Das Ergebnis ist in Fig. 1B gezeigt. Dabei zeigt sich, dass die Prostatakarzinomzellen deutlich gefärbt, die Kolonkarzinomzellen jedoch praktisch ungefärbt bleiben. Die erfindungsgemäße Verbindung wird folglich von den Prostatakarzinomzellen, nicht aber von den Kolonkarzinomzellen, in das Zytoplasma aufgenommen.

In einem weiteren Experiment wurden Prostatakarzinomzellen (PC-3) und Osteosarkomzellen (U2OS) 20 Minuten mit Gd-DOTA-Lys(FITC)-Aminoundecanal (Verbindung 10) inkubiert und mittels CLSM analysiert.

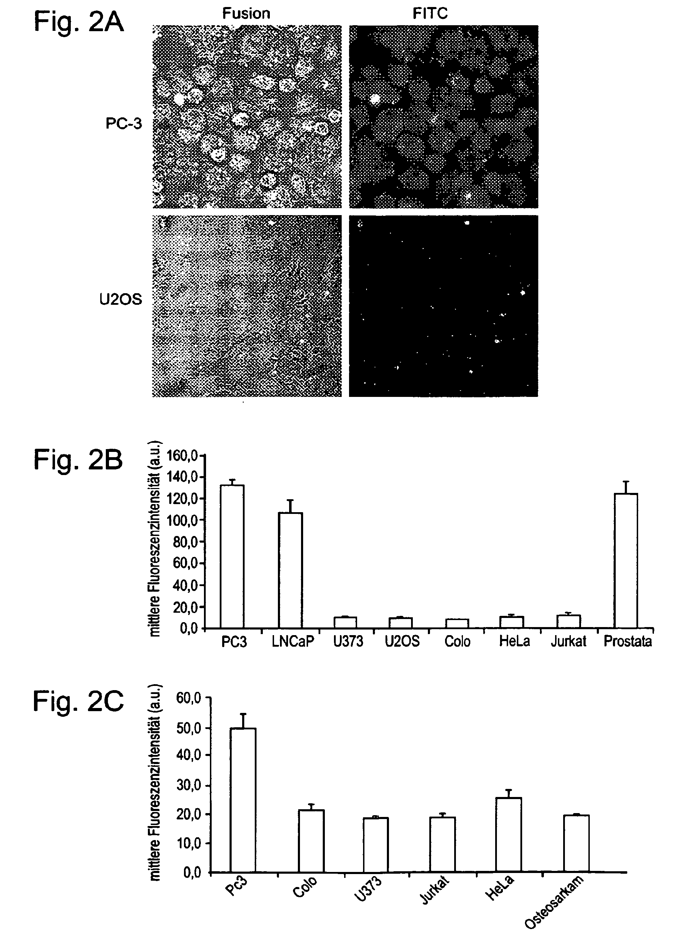

Das Ergebnis ist in Fig. 2A gezeigt. Die erfindungsgemäße Verbindung wird demnach sehr gut in das Zytoplasma der Prostatakarzinomzellen, nicht aber in Osteosarkomzellen, aufgenommen. FITC allein (ohne Undecanal) wird von den Zellen nicht aufgenommen (nicht gezeigt).

Als nächstes wurden sieben Tumorzelllinien sowie gesunde Prostatazellen (CC2555) mit Gd-DOTA-Lys(FITC)-Aminoundecanal (Verbindung 10) 20 Minuten inkubiert und mittels Fluoreszenz-aktivierter Zellsortierung (FACS) analysiert.

Das Ergebnis ist in Fig. 2B dargestellt. Hierbei zeigt sich, dass die beiden Prostatakarzinomzelllinien (PC-3, LNCaP) und die gesunden Prostatazellen (CC2555), nicht aber die Nicht-Prostatatumorzelllinien, gefärbt werden. Demnach nehmen lediglich die Prostatazellen die erfindungsgemäße Verbindung auf.

In einem weiteren Experiment wurden verschiedene Karzinomzellen 20 Minuten mit Sulforhodaminsulfonamido-β-Ala-Aminooctanal (Verbindung 4) inkubiert und mittels FACS analysiert.

Das Ergebnis ist in Fig. 2C dargestellt. Dabei zeigt sich, dass die Prostatakarzinomzellen (PC3) die höchste Fluoreszenz aufweisen, d.h. die erfindungsgemäße Verbindung aufnehmen. Die übrigen Karzinomzelllinien zeigen lediglich eine geringe Färbung, nehmen also die erfindungsgemäße Verbindung nur in sehr geringem Maße auf.

In einem weiteren Ansatz wurden U373-Gliomzellen und PC3-Prostatakarzinomzellen mit Lys(FITC)-Aminobourgeonal (Verbindung 7) für 48 Stunden inkubiert und mittels CLSM analysiert.

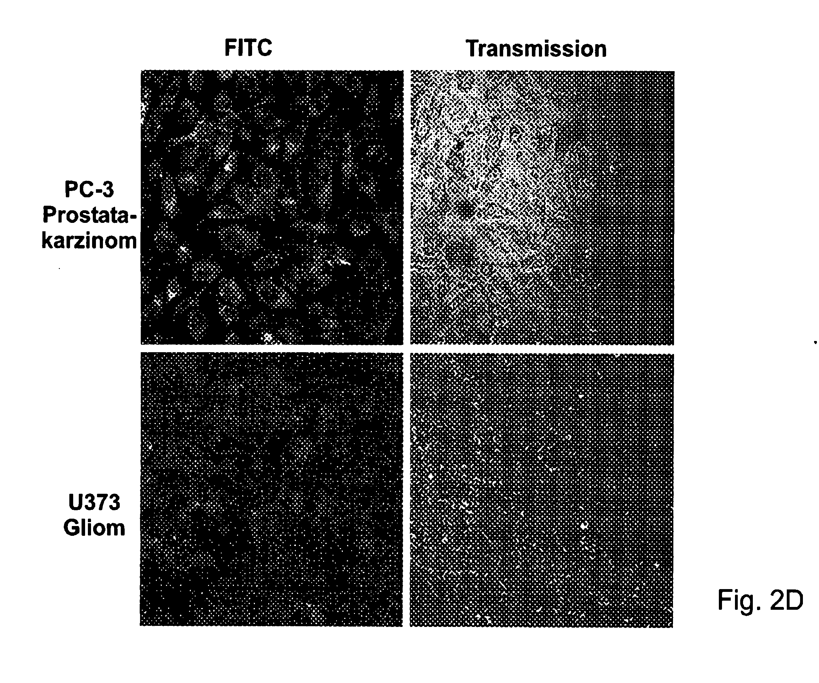

Das Ergebnis ist in Fig. 2D dargestellt. Die Prostatakarzinomzellen, jedoch nicht die Gliomzellen werden zytoplasmatisch gefärbt. Wie bei den anderen Konjugaten zeigen sich keine zytotoxischen Veränderungen.

In einem weiteren Experiment wurden LNCaP-Zellen 20 Minuten mit Gd-DOTA allein (DOTAREM, Guerbet, Frankreich) und mit der erfindungsgemäßen Verbindung 10 Gd-DOTA-Lys(FITC)-Aminoundecanal in einem Eppendorfröhrchen inkubiert und ausgewaschen.

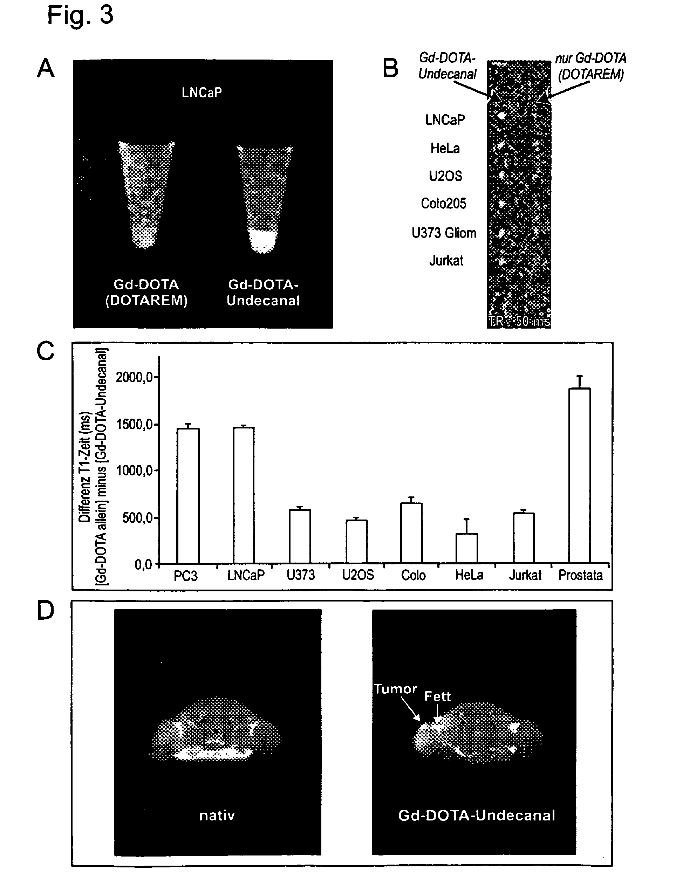

Das Ergebnis ist in Fig. 3A dargestellt. Dabei zeigt sich, dass die einfache Gd-DOTA-Verbindung von den Prostatakarzinomzellen nicht aufgenommen wird. Nach der Inkubation und dem Auswaschen bleiben die Zellen daher dunkel; vgl. Fig. 3A, links. Die erfindungsgemäße Gd-DOTA-Undecanalverbindung wird aber über den Maiglöckchenduftrezeptor in das Zytoplasma der Prostatakarzinomzellen aufgenommen. Nach Auswaschen und Zentrifugation sind diese Zellen daher hell; vgl. Fig. 3A, rechts.

### 2.2 Einfluss der Kopplung von Undecanal an Gd-DOTA

Sechs verschiedene Tumorzelllinien (darunter LNCaP-Prostatakarzinomzellen) wurden 20 Minuten einerseits mit Gd-DOTA allein (DOTAREM, Guerbet, Frankreich) und andererseits mit der erfindungsgemäßen Verbindung 10 Gd-DOTA-Lys(FITC)-Aminoundecanal in Eppendorfröhrchen inkubiert und anschließend dreimal mit PBS ausgewaschen.

Das Ergebnis ist in Fig. 3B dargestellt. Die Eppendorfröhrchen sind quer angeschnitten. In der rechten Reihe bleiben sowohl die Prostatakarzinomzellen (LNCaP) als auch die anderen Karzinomzellen nach Inkubation und Auswaschen mit der einfachen Gd-DOTA-Verbindung dunkel. Erst nach Inkubation mit der erfindungsgemäßen Verbindung 10 Gd-DOTA-Lys(FITC)-Aminoundecanal kommt es zu einer Erhöhung der Signalintensität, und zwar nur bei LNCaP-Prostatakarzinomzellen; vgl. Fig. 3B, linke Spalte, oben.

Die Kopplung von Undecanal an Gd-DOTA (linke Spalte) bewirkt also im Vergleich zu einfachem Gd-DOTA (rechte Spalte) eine deutlich stärkere T1-Zeit-Verkürzung bei Prostatakarzinomzellen, nicht aber bei den anderen Tumorzelllinien. Die T1-Zeit-Verkürzung ist ein Maß für die Zunahme der Helligkeit (Signalintensität) in den Zellen, welche durch die zelluläre Aufnahme einer Gadoliniumverbindung in den T1-gewichteten Aufnahmen verursacht wird.

In Fig. 3C geben die Balken die Differenz der T1-Zeit der jeweiligen Karzinomzellen und der gesunden Prostatazellen (CC2555) nach Inkubation mit der erfindungsgemäßen Verbindung 10 Gd-DOTA-Lys(FITC)-Aminoundecanal und der einfachen Gd-DOTA-Verbindung an. Die Differenz der T1-Zeit ist ein Maß dafür, inwieweit die Verbindung bei der jeweiligen Zelllinie zu einer Verkürzung der T1-Zeit (Zunahme der Helligkeit, Signalintensität) führt. Diese Einheit wird verwendet, da die Zelllinien unterschiedliche T1-Zeiten vor der Inkubation haben. Differenz der T1-Zeit = (T1-Zeit vor Inkubation) minus (T1-Zeit nach Inkubation). Die T1-Zeit vor der Inkubation ist länger als die nach der Inkubation. Je kürzer die T1-Zeit, desto heller die Zellen.

Wie der Abbildung Fig. 3C zu entnehmen ist, kommt es bei den PC-3 und LNCaP-Prostatakarzinomzellen sowie bei den gesunden Prostatazellen (CC2555) zur stärksten Zunahme der Signalintensität in den T1-gewichteten MRT-Aufnahmen (stärkste Verkürzung der T1-Zeit, größte Differenz von der T1-Zeit vor und nach Inkubation, dies in Relation zum Ausgangshelligkeitswert vor Inkubation).

### 2.3 Selektive Aufnahme der erfindungsgemäßen Verbindungen in das Prostatakarzinom in vivo

In einem weiteren Versuch an einer Nacktmaus, in die ein humanes PC3-Prostatakarzinom in die rechte Flanke implantiert wurde, wurde 10 Tage nach der Implantation die erfindungsgemäße Verbindung 10 Gd-DOTA-Lys(FITC)-Aminoundecanal intravenös appliziert.

Das Ergebnis ist in Fig. 3D vor und 90 Minuten nach intravenöser Gabe dargestellt. Dabei zeigt sich, dass diese ausschließlich in das Zytoplasma der Prostatakarzinomzellen aufgenommen wird, welche sich dann mit erhöhter Signalintensität darstellen. Nicht-Prostatakarzinomzellen bleiben ungefärbt, nehmen also die erfindungsgemäße Verbindung nicht auf.

In einem weiteren Experiment wurden Organe von Nacktmäusen, in die ein humanes Prostatakarzinom (PC-3) implantiert wurde, 90 Minuten nach intraperitonealer Injektion der erfindungsgemäßen Verbindung 1 Sulforhodaminsulfonamido-Aminoundecanal anhand von Gefrierschnitten mittels CLSM analysiert.

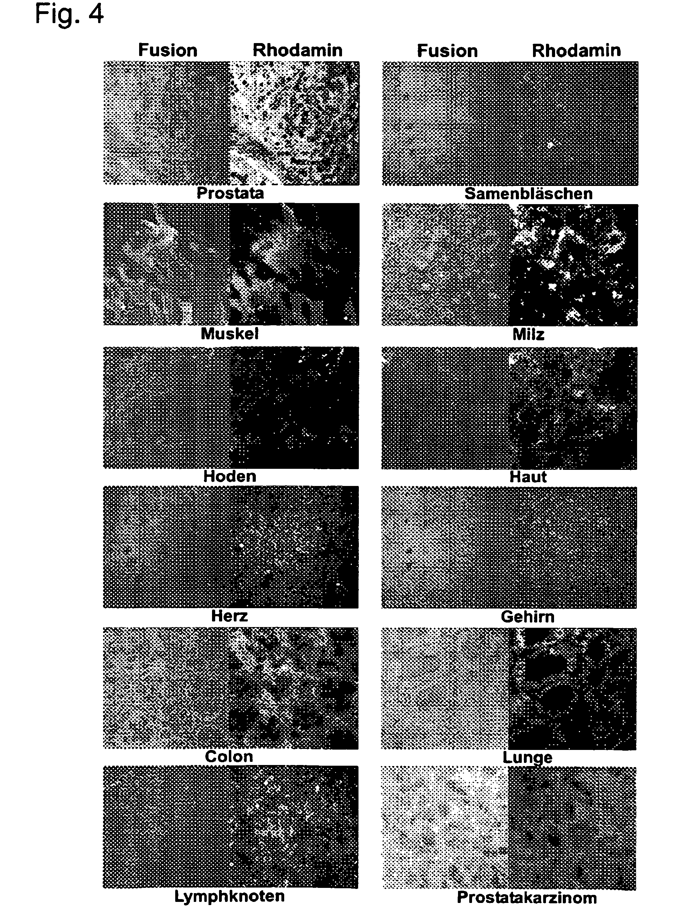

Das Ergebnis ist in Fig. 4 dargestellt. Dabei zeigt sich, dass im Vergleich zu möglichen Metastasierungsorten wie Lymphknoten und Lunge das Prostatakarzinom eine deutlich stärkere Rotfluoreszenz aufweist. Interessanterweise zeigt sich auch hier in gesundem Prostatagewebe kaum eine Färbung. Die erfindungsgemäße Verbindung wird demnach nahezu ausschließlich von Prostatakarzinomzellen aufgenommen.

In einem weiteren Experiment wurden Gefrierschnitten des in Nacktmäuse implantierten Prostatakarzinoms (PC-3) 20 und 180 Minuten nach intraperitonealer Injektion der erfindungsgemäßen Verbindung 1 Sulforhodaminsulfonamido-Aminoundecanal untersucht.

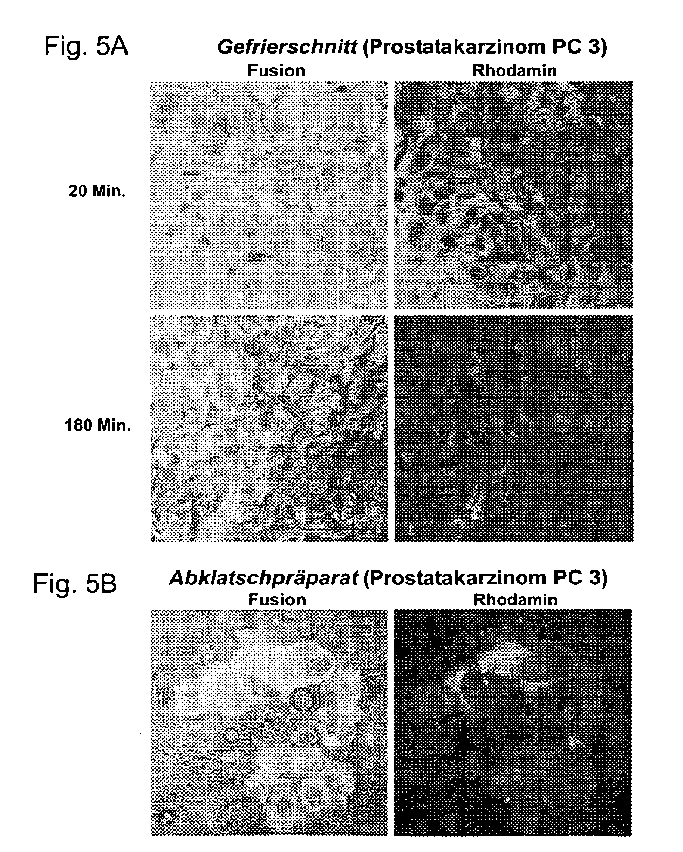

Das Ergebnis ist in Fig. 5A dargestellt. Dabei zeigt sich, dass die Prostatakarzinomzellen bereits nach 20 Minuten die erfindungsgemäße Verbindung sehr gut in das Zytoplasma aufgenommen haben. Nach 180 Minuten jedoch kommt es zu einem Efflux der Verbindung aus dem Zytoplasma der Prostatakarzinomzellen. Dieses Ausschleusen erklärt, dass die erfindungsgemäße Verbindung von den Tieren sehr gut vertragen wird.

In Fig. 5B sind "Abklatschpräparate" (engl.: "Touch Prints") des PC-3-Prostatakarzinoms 20 Minuten nach intraperitonealer Injektion dargestellt. Die Zellen eines frisch angeschnittenen Tumors werden hierbei auf einem Objektträger ausgestrichen. Damit können Gefrierartefakte oder Artefakte durch Fixierung mittels Formalin ausgeschlossen werden. Dieses Experiment bestätigt demnach, dass bereits 20 Minuten nach intraperitonealer Injektion die erfindungsgemäße Verbindung das Zytoplasma der Prostatakarzinomzellen erreicht hat.

### 3. Schlussfolgerung

Mit der vorliegenden Erfindung wird erstmals ein Mittel zum direkten und zuverlässigen Nachweis von Prostatazellen bzw. Prostatakarzinomzellen bereitgestellt. Dieses Mittel weist zumindest einen Liganden des Maiglöckchenduftrezeptors (OR17-4 bzw. hOR17-4) auf, optional einen mittels bildgebender Verfahren detektierbaren Marker. Exemplarisch wird die Erfindung anhand von fünf verschiedenen Verbindungen demonstriert.

## Patentansprüche

1. Verwendung eines Liganden des Maiglöckchenduftrezeptors (OR17-4) zur
Herstellung eines Mittels zur Identifizierung von Prostatazellen.

2. Verwendung nach Anspruch 1, **dadurch gekennzeichnet, dass** es sich bei dem Maiglöckchenduftrezeptor um die humane Variante hOR17-4 handelt.

3. Verwendung nach Anspruch 1 oder 2, **dadurch gekennzeichnet, dass** der Ligand ausgewählt ist aus der Gruppe bestehend aus: Undecanal, 3-phenyl-2-Propenal (Zimtaldehyd), Octanal, Bourgeonal, Benzaldehyd, Phenylacetaldehyd, 3-Phenylpropanal, 3-Phenylbutyraldehyd, 4-Phenylbutyraldehyd, Canthoxal, Cyclamal, Floralazon, Lilial, (4-tert-butylphenoxy)acetaldehyd, p-Anisyl-Format, Piperonylacetat, Zimtalkohol, Hydrozimtaldehyd, Methylzimtaldehyd, Methyl-Hydro-Zimtaldehyd, Methylnonylaldehyd, Phenylethanol, Phenylpropanol, Methylzimtaldehyd, Isobutyraldehyd, Helional, Lyral, Methyl-Phenyl-Pentanal, Cyclosal, Foliaver, Trimethylmethan, Trifernal, Piperonal, Isovaleraldehyd, (4-Hydroxyphenyl)-Butan-2-on (Himbeerketon), Vanillin, Safrol, Heptanal, Anethol, 5-Phenylvaleraldehyd, Isopropylbenzen, 3-Phenylbutylalkohol und 3-Phenylpropionsäure.

4. Verwendung nach einem der vorherigen Ansprüche, **dadurch gekennzeichnet, dass** das Mittel ferner einen mittels bildgebender Verfahren detektierbaren Marker aufweist, der vorzugsweise mittels Fluoreszenzmikroskopie detektierbar ist und weiter vorzugsweise ausgewählt ist aus: Fluorescein-Isothiocyanat (FITC), Rhodamin, Rhodamin Isothiocyanat (RITC), Sulforhodamin B, Dansylchlorid, Fluorescamin, grünfluoreszierendes Protein (GFP), Ethidiumbromid, 4',6-Diamidino-2-phenylindol (DAPI), Coumarin, Luciferase, Phycoerythrin (PE), Cy2, Cy3.5, Cy5, Cy 7, Texas Red, Alexa Fluor, Fluor X, Red 613, BODIPY-FL, TRITC, DS Red, GFP, DS Red.

5. Verwendung nach Anspruch 4, **dadurch gekennzeichnet, dass** der Marker mittels Magnetresonanztomographie (MRT) dektektierbar ist und vorzugsweise ein Komplexbildner für Metalle sowie ein komplexiertes Metall, einschließlich Gadolinium (Gd), Europium (Eu), Gallium (Ga), Mangan (Mn), Eisen (Fe), Yttrium (Y) sowie deren Isotope, aufweist, wobei weiter vorzugsweise der Komplexbildner für Metalle ausgewählt ist aus der Gruppe bestehend aus: Tetraazacyclododecantetraessigsäure (DOTA), Diethylentriaminpentaessigsäure (DTPA), BOPTA, EOB-DTPA, DTPA-DMA, HP-DOBA, DTPA-BMEA, HIDA, DTDP, Porphyrine, Texaphyrine, TEKES, Fullerene, Kronenether.

6. Verwendung nach Anspruch 4 oder 5, **dadurch gekennzeichnet, dass** der Marker mittels Computertomographie detektierbar ist und vorzugsweise eine Iodverbindung, einschließlich lopromid, Thyroxin, Triiodothyronin und Triiodobenzoesäure, aufweist.

7. Verwendung nach einem der Ansprüche 4 bis 6, **dadurch gekennzeichnet, dass** der Marker mittels nuklearmedizinischer Verfahren detektierbar ist und vorzugsweise einen γ-Strahler, einschließlich Gadolinium-153, oder einen β-Strahler, einschließlich Sr-89, Y-90, I-131, Er-169, Re-186 und Re-188, aufweist.

8. Verwendung nach Anspruch 4, **dadurch gekennzeichnet, dass** das Mittel eine Verbindung aufweist, die ausgewählt ist aus der Gruppe bestehend aus: Sulforhodaminsulfonamido-Aminoundecanal, Sulforhodaminsulfonamido-βAla-Aminozimtaldehyd, Sulforhodaminsulfonamido-Aminobenzaldehyd, Sulforhodaminsulfonamido-β-Ala-Aminooctanal, Sulforhodaminsulfonamido-ßAla-Aminoundecanal, FITC-ßAla-Aminoundecanal und Lys(FITC)-Aminobourgeonal.

9. Verwendung nach Anspruch 5, **dadurch gekennzeichnet, dass** das Mittel eine Verbindung aufweist, die ausgewählt ist aus der Gruppe bestehend aus: Gd-DOTA-Aminoundecanal, Gd-DOTA-ßAla-Aminoundecanal.

10. Verwendung nach einem der Anspruch 4 oder 5, **dadurch gekennzeichnet, dass** das Mittel eine Verbindung aufweist, die ausgewählt ist aus der Gruppe bestehend aus: Gd-DOTA-Lys(FITC)-Aminoundecanal, Gd-DOTA-ßAla-Lys(FITC)-Aminoundecanal, FITC-ßAla-Lys(Gd-DOTA)-Aminoundecanal, Gd-DOTA-ßAla Lys(FITC)-IPLVVPL-Aminoundecanal und Gd-DOTA(4)-Lys(4)-Lys(2)-Lys(FITC)-Aminoundecanal.

11. Verwendung nach einem der vorherigen Ansprüche, **dadurch gekennzeichnet, dass** es sich bei dem Mittel um ein Diagnostikum zum Nachweis von Prostatakarzinomen vorzugsweise von Prostatakarzinommetastase, weiter vorzugsweise um solche in den Lymphknoten und/oder Knochen handelt.

12. Verwendung eines Liganden des Maiglöckchenduftrezeptors (OR17-4) zur Herstellung eines Therapeutikums gegen Prostatakarzinome, das ein Zytostatikum und den Liganden des Maiglöckchenduftrezeptors (OR17-4) aufweist.

13. Verwendung nach Anspruch 12, **dadurch gekennzeichnet, dass** das Therapeutikum ein Zytostatikum aufweist, das vorzugsweise ausgewählt ist aus der Gruppe bestehend aus: Alkylantien, Platinanaloga, Interkalantien, Antibiotika, Mitosehemmer, Texane, Topoisomerasehemmer und Antimetabolite.

## Claims

1. Use of a ligand of the lilly-of-the-valley odorant receptor (OR17-4) for manufacturing a composition for identifying prostate cells.

2. Use of claim 1, **characterized in that** the lilly-of-the-valley odorant receptor is the human variant hOR17-4.

3. Use of claim 1 or 2, **characterized in that** the ligand is selected from the group consisting of: undecanal, 3-phenyl-2-propenal (cinnamaldehyde), octanal, bourgeonal, benzaldehyde, phenylacetaldehyde, 3-phenylpropanal, 3-phenylbutylradehyde, 4-phenylbutyraldehyde, canthoxal, cyclamal, floralazone, lilial, (4-terbutylphenoxy)acetaldehyde, p-anisyl-formate, piperonylacetate, cinnamyl alcohol, hydrocinnamaldehyde, methylcinnamaldehyde, methyl-hydro-cinnamaldehyde, methylnonylaldehyde, phenetyl alcohol, phenylpropanol, methylcinnamaldehyde, isobutyraldehyde, helional, lyral, methyl-phenyl-pentanal, cyclosal, foliaver, trimethylmethane, trifernal, piperonal, isovaleraldehyde, (4-hydroxyphenyl)-butan-2-on (raspberry ketone), vanillin, safrole, heptanal, anethole, 5-phenylvalder-aldehyde, isopropylbenzene, 3-phenylbutyl alcohol and 3-phenylpropionic acid.

4. Use of any of the preceding claims, **characterized in that** the composition further comprises a marker detectable by means of an imaging method, preferably detectable by means of fluorescence microscopy, and further preferably selected from fluorescein isothiocyanate (FITC), rhodamine, rhodamine isothiocyanate (RITC), sulforhodamine B, dansyl chloride, fluorescamine, green fluorescent protein (GFP), ethidium bromide, 4'-6-diamidino-2-phenylindole (DAPI), coumarin, luciferase, phycoerythrin (PE), Cy2, Cy3.5, Cy5, Cy7, texas red, alexa fluor, fluor X, red 613, BODIPY-FL, TRITC, DS red, GFP, DS red.

5. Use of claim 4, **characterized in that** the marker is detectable by means of magnetic resonance tomography (MRT) and preferably comprises a complexing agent for metals and a complexed metal, including gadolinium (Gd), europium (Eu), gallium (Ga), manganese (Mn) iron (Fe), yttrium (Y) and its isotopes, wherein further preferably the complexing agent for metals is selected from the group consisting of: tetraazacyclododecane tetraacetic acid (DOTA), diethylenetriaminepentaacetic acid (DTPA), BOPTA, EOB-DTPA, DTPA-DMA, HP-DOBA, DTPA-BMEA, HIDA, DTDP, porphyrine, texaphyrine, TEKES, Fullerene, crown ether.

6. Use of any of claims 4 or 5, **characterized in that** the marker is detectable via computer tomography and preferably comprises an iodine compound, including iopromide, thyroxine, triiodothyronine and triiodobenzoic acid.

7. Use of any of claims 4 to 6, **characterized in that** the marker is detectable by means of nuclear medical methods and preferably comprises a γ-radiator, including gadolinium-153 or a β-radiator, including Sr-89, Y-90, I-131, Er-169, Re-186 and Re-188.

8. Use of claim 4, **characterized in that** the composition comprises a compound selected from the group consisting of: sulforhodaminesulfonamido aminoundecanal, sulforhodaminesulfonamido-βala-aminocinnamaldehyde, sulforhodaminesulfonamido-aminobenzaldehyde, sulforhodaminesulfonamido-βala-aminooctanal, sulforhodaminesulfonamido-βala-aminoundecanal, FITC-βala-aminoundecanal and lys(FITC)-aminobourgeonal.

9. Use of claim 5, **characterized in that** the composition comprises a compound selected from the group consisting of: Gd-DOTA-aminoundecanal, Gd-DOTA-βala-aminoundecanal.

10. Use of any of claims 4 or 5, **characterized in that** the composition comprises a compound selected from the group consisting of: Gd-DOTA-lys(FITC)-aminoundecanal, Gd-DOTA-βala-lys(FITC)-aminoundecanal, FITC-βala-lys(Gd-DOTA)-aminoundecanal, Gd-DOTA-βala-lys(FITC)-IPLVVPL-aminoundecanal and Gd-DOTA(4)-lys(4)-lys(2)-lys(FITC)-aminoundecanal.

11. Use of any of the preceding claims, **characterized in that** the composition is a diagnostic composition for the detection of prostate carcinoma, preferably of prostate carcinoma metastases, further preferably such in the lymph knots and/or bones.

12. Use of a ligand of the lilly-of-the-valley odorant receptor (OR17-4) for manufacturing a therapeutic composition against prostate carcinoma comprising a cytostatic agent and the ligand of the lilly-of-the-valley odorant receptor (OR17-4).

13. Use of claim 12, **characterized in that** the therapeutic composition comprises a cytostatic agent, preferably selected from the group consisting of: alkylating agents, platinium analogues, intercalating agents, antibiotics, mitosis inhibitors, texanes, topoisomerase inhibitors and antimetabolites.

## Revendications

1. Utilisation d'un ligand du récepteur du parfum du muguet (OR17-4) pour la fabrication d'un moyen d'identification de cellules de la prostate.

2. Utilisation selon la revendication 1, **caractérisée en ce que** le récepteur du parfum du muguet est la variante humaine hOR17-4.

3. Utilisation selon la revendication 1 ou 2, **caractérisée en ce que** le ligand est choisi dans le groupel constitué par : l'undécanal, le 3-phényl-2-propénal (cinnamaldéhyde), l'octanal, le bourgeonal, le benzaldéhyde, le phénylacétaldéhyde, le 3-phénylpropanal, le 3-phénylbutyraldéhyde, le 4-phénylbutyraldéhyde, le canthoxal, le cyclamal, la floralazone, le lilial, le (4-tert-butylphénoxy)acétaldéhyde, le formate de p-anisyle, l'acétate de pipéronyle, l'alcool cinnamique, l'hydrocinnamaldéhyde, le méthylcinnamaldéhyde, le méthyl-hydro-cinnamaidéhyde, le méthylnonylaldéhyde, le phényléthanol, le phénylpropanol, le méthylcinnamaidéhyde, l'isobutyraldéhyde, l'hélional, le lyral, le méthyl-phényl-pentanal, le cyclosal, le Foliaver, le triméthylméthane, le trifernal, le pipéronal, l'isovaléraldéhyde, la (4-hydroxyphényl)-butan-2-one (cétone de framboise), la vanilline, le safrol, l'heptanal, l'anéthol, le 5-phénylvaléraldéhyde, l'isopropylbenzène, l'alcool 3-phénylbutylique et l'acide 3-phénylpropionique.

4. Utilisation selon l'une quelconque des revendications précédentes, **caractérisée en ce que** le moyen comprend également un marqueur détectable par un procédé d'imagerie, qui est de préférence détectable par microscopie à fluorescence et également de préférence choisi parmi : l'isothiocyanate de fluorescéine (FITC), la rhodamine, l'isothiocyanate de rhodamine (RITC), la sulforhodamine B, le chlorure de dansyle, la fluorescamine, la protéine fluorescente verte (GFP), le bromure d'éthidium, le 4',6-diamidino-2-phénylindol (DAPI), la coumarine, la luciférase, la phycoérythrine (PE), Cy2, Cy3.5, Cy5, Cy7, Texas Red, Alexa Fluor, Fluor X, Red 613, BODIPY-FL, TRITC, DS Red, GFP, DS Red.

5. Utilisation selon la revendication 4, **caractérisée en ce que** le marqueur est détectable par tomographie par résonance magnétique (TRM) et comprend de préférence un complexant pour métaux en particulier un métal complexé, en particulier le gadolinium (Gd), l'europium (Eu), le gallium (Ga), le manganèse (Mn), le fer (Fe), l'yttrium (Y) et leurs isotopes, le complexant pour métaux étant également de préférence choisi dans le groupe constitué par : l'acide tétraazacyciododécanetétraacétique (DOTA), l'acide diéthylènetriaminepentaacétique (DTPA), BOPTA, EOB-DTPA, DTPA-DMA, HP-DOBA, DTPA-BMEA, HIDA, DTDP, la porphyrine, la texaphyrine, TEKES, les fullerènes, les éthers couronnes.

6. Utilisation selon la revendication 4 ou 5, **caractérisée en ce que** le marqueur est détectable par tomodensitométrie et comprend de préférence un composé d'iode, y compris l'iopromide, la thyroxine, la triiodothyronine et l'acide triiodobenzoïque.

7. Utilisation selon l'une quelconque des revendications 4 à 6, **caractérisée en ce que** le marqueur est détectable par un procédé nucléaire médical et comprend de préférence un émetteur gamma γ, y compris le gadolinium-153, ou un émetteur béta β, y compris Sr-89, Y-90, I-131, Er-169, Re-186 et Re-188.

8. Utilisation selon la revendication 4, **caractérisée en ce que** le moyen comprend un composé qui est choisi dans le groupe constitué par : le sulforhodaminesulfonamido-aminoundécanal, le sulforhodaminesulfonamido-βAla-aminocinnamaldéhyde, le sulforhodaminesulfonamido-aminobenzaldéhyde, le sulforhodaminesulfonamido-β-Ala-aminooctanal, le sulforhodaminesulfonamid-βAla-aminoundécanal, le FITC-βAla-aminoundécanal et le Lys(FITC)-aminobourgeonal.

9. Utilisation selon la revendication 5, **caractérisée en ce que** le moyen comprend un composé qui est choisi dans le groupe constitué par : le Gd-DOTA-aminoundécanal, le Gd-DOTA-βAla-aminoundécanal.

10. Utilisation selon l'une quelconque des revendications 4 ou 5, **caractérisée en ce que** le moyen comprend un composé qui est choisi dans le groupe constitué par : le Gd-DOTA-Lys(FITC)-aminoundécanal, le Gd-DOTA-βAla-Lys(FITC)-aminoundécanal, le FITC-βAla-Lys(Gd-DOTA)-aminoundécanal, le Gd-DOTA-βAla-Lys(FITC)-IPLWPL-aminoundécanal et le Gd-DOTA(₄)-Lys(4)-Lys(2) Lys(FITC)-aminoundécanal.

11. Utilisation selon l'une quelconque des revendications précédentes, **caractérisée en ce que** le moyen est un agent de diagnostic pour la détection de carcinomes de la prostate, de préférence d'une métastase de carcinomes de la prostate, de manière davantage préférée de ceux-ci dans les ganglions lymphatiques et/ou les os.

12. Utilisation d'un ligand du récepteur du parfum du muguet (OR17-4) pour la fabrication d'un agent thérapeutique contre les carcinomes de la prostate, qui comprend un agent cytostatique et le ligand du récepteur du parfum du muguet (OR17-4).

13. Utilisation selon la revendication 12, **caractérisée en ce que** l'agent thérapeutique comprend un agent cytostatique qui est de préférence choisi dans le groupe constitué par : les alkylants, les analogues de platine, les intercalants, les antibiotiques, les inhibiteurs de mitose, les texanes, les inhibiteurs de topoisomérase et les antimétabolites.
